# EUROPEAN PATENT APPLICATION

(11) **EP 3 865 141 A1**
(43) Date of publication of application: **18.08.2021**
(21) Application number: 19871275.4
(22) Date of filing: 07.10.2019
(51) Int. Cl.: A61K 35/76, A61K 31/739, A61K 35/741, A61K 38/19, A61K 39/395, A61K 45/00, A61K 48/00, A61P 35/00, A61P 43/00, C07K 14/115, C07K 14/715, C12N 7/04, C12N 15/12

(54) **ANTICANCER AGENT, PHARMACEUTICAL COMPOSITION FOR CANCER TREATMENT, AND KIT**

(30) Priority: 09.10.2018 JP 2018191262; 22.02.2019 JP 2019030897
(71) Applicant: Biocomo Incorporation, Mie, 510-1233 (JP); Mie University, Tsu-shi, Mie 514-8507 (JP)
(72) Inventor: FUKUMURA Masayuki, Mie-gun, Mie 510-1233 (JP); OHTSUKA Junpei, Mie-gun, Mie 510-1233 (JP); NOSAKA Tetsuya, Tsu-shi, Mie 514-8507 (JP)
(74) Representative: Nederlandsch Octrooibureau
(86) International application number: PCT/JP2019/039487
(87) International publication number: WO 2020/075672

(57) **Abstract**

An anticancer agent contains a virus presenting a desired protein or peptide on a virus particle envelope, as an active ingredient.

## Description

### [Technical Field]

The present invention relates to an anticancer agent, a pharmaceutical compositions for cancer treatment, and a kit.

Priorities are claimed on Japanese Patent Application No. 2018-191262 filed on October 9, 2018 and Japanese Patent Application No. 2019-030897 filed on February 22, 2019, the contents of which are incorporated herein.

### [Background Art]

Cancer cells may proliferate partly due to enhancement of immunosuppressive mechanisms such as PD-1/PD-L1. Immune checkpoint inhibitors such as an anti-PD-1 antibody inhibit the enhanced negative immune escape mechanism and suppress the proliferation of cancer cells (see, for example, Patent Document 1).

### [Citation List]

### [Patent Documents]

[Patent Document 1]
   Japanese Patent No. 5159730
[Patent Document 2]
   PCT International Publication No. WO2016/199936
[Patent Document 3]
   Japanese Unexamined Patent Application, First Publication No. 2016-102113

### [Non Patent Documents]

[Non Patent Document 1]
   BMC Cancer, (2018) 18:425
[Non Patent Document 2]
   Clin. Cancer Res., (2017): 23 (3), 707
[Non Patent Document 3]
   Eur. J. Immunol., (2002) 32: 3617
[Non Patent Document 4]
   Sci. Rep., (2018) 8: 2278
[Non Patent Document 5]
   Transplantation Proceedings, (2016) 48: 1270
[Non Patent Document 6]
   Clin Cancer Res., (2018) 24 (8): 1816
[Non Patent Document 7]
   JEADV, (2017) 31: 1324
[Non Patent Document 8]
   Cancer Res., (2013) 73: 7189
[Non Patent Document 9]
   Sci. Transl. Med., (2018) 10 (426), eaan4488.
[Non Patent Document 10]
   Gene Ther., (2014) 21 (8): 775
[Non Patent Document 11]
   Hum Gene Ther., (2013) 24 (7): 683
[Non Patent Document 12]
   Front. Immunol., (2013) 4: 11
[Non Patent Document 13]
   Blood. (2014) 123 (14): 2172
[Non Patent Document 14]
   Nat Commun., (2018) 9: 4679
[Non Patent Document 15]
   Clin. Cancer Res., (2017) 23: 1929
[Non Patent Document 16]
   Nat Commun., (2019) 10: 2141
[Non Patent Document 17]
   FEBS J., (2008) 275: 2296
[Non Patent Document 18]
   J. Immunol., (2009) 183 (3): 1851

### [Summary of Invention]

### [Technical Problem]

However, many types of cancer are difficult to suppress the proliferation only with immune checkpoint inhibitors that inhibit negative immunity. Therefore, development of antitumor immune activators that activate dendritic cells, activate positive immune systems actively attacking cancer cells, that is, activate natural killer cells (NK cells), CD4-positive helper T cells, and cytotoxic T cells (CTL), and suppress the proliferation of cancer cells has been promoted.

The molecules of CD134 (OX40), CD137 (4-1BB), GITR, CD27, CD30, and the like of TNF receptor superfamily (TNFRSF, hereinafter, referred to as a TNFSF receptor particularly in a case of indicating a receptor and referred to as a TNFSF ligand in a case of indicating a ligand) expressed in T cells, NK cells, and the like exhibit antitumor immune effects such as CTL by activating positive immunity. Since distant metastasis of advanced solid cancer occurs frequently, patients who are difficult to treat with methods of the conventional art are treated by intratumoral administration of oncolytic virus or the like, and the possibility that systemically induced antitumor immune effects are also exerted in distant cancer has been reported.

That is, it is considered that administration of a drug to solid cancer enhances systemic antitumor immune effects such as CTL, and thus distant cancer separated from the administration site can be suppressed. For that purpose, the functions of NK cells and T cells are enhanced by activation of dendritic cells and signal activation of TNFRSF such as CD134 (OX40), CD137 (4-1BB), GITR, CD27, CD30, or the like, and thus antitumor effects on distant cancer can be achieved.

A TNFSF ligand protein expressed in immune cells in vivo stimulates these receptors expressed in T cells and the like. The ligand protein forms a trimer and binds to a receptor (also referred to as a receptor or R) protein to efficiently transmit a signal. However, since the signal cannot be efficiently transmitted only with the ligand protein, it is not easy to use the ligand protein as a protein preparation. Therefore, agonist antibodies that bind to receptors and efficiently transmit signals have been exclusively produced and used.

As an antibody drug, for example, the anti-CD20 antibody (rituximab) in malignant lymphoma that highly expresses CD20 is an antibody drug which directly binds to CD20-positive cancer cells so that the Fc site of the antibody binds to macrophages, NK cells, and the like that express FcR and thus damages cancer cells by antibody-dependent cellular cytotoxicity (ADCC activity), complement-dependent cellular cytotoxicity (CDC activity), and the like. Many types of antibodies that directly bind to cancer cells and suppress tumors are commercially available and make great profits.

Further, immune checkpoint inhibitory antibodies (nivolumab and the like) block negative immune signals by inhibiting the binding between PD-1 and PD-L1/L2 and activate antitumor immunity such as CTL. The immune checkpoint inhibitory antibodies are already commercially available and make great profits.

Meanwhile, the agonist antibodies that activate the TNFRSF signals exhibited high antitumor effects in nonclinical tests by systemic administration, but high hepatotoxicity was reported in clinical tests of anti-4-1BB agonist antibodies while the effectiveness was shown (see Non Patent Document 15), and problems of temporal interruption of the tests occurred. Therefore, the agonist antibodies have not been commercially available as an antibody drug.

Further, even in animal tests performed using GITR agonist antibodies (DTA-1), anaphylactic shock due to overdose was reported (see Non Patent Document 13).

Agonist antibodies are capable of independently binding to the TNFSF receptors and transmitting signals. The receptor has four cysteine-rich domains (CRD). The CRD domains to which the agonist antibodies bind vary, and this results in a difference in signal transmission intensity. It is considered that the toxicity varies depending on the intensity (see Non Patent Document 14).

In addition, human antibodies have 6 types of IgG subclasses. These Fc regions have a property of binding to activated FcγR present on the surfaces of immune cells and the like and activating signals through the ITAM motif, a property of conversely binding to inhibitory FcyRIIB on the surface of each cell and suppressing signals through the ITIM motif, or a property of not binding to FcR. A human IgG1 antibody binds to activated FcyR and enhances ADCC/CDC activity, and human IgG4 is considered not to bind to FcR.

Meanwhile, it has also been reported that antitumor immunity using TNFRSF signals does not function at all in KO mice with inhibitory FcyRIIB. Urelumab, an anti-4-1BB agonist antibody that has been reported to be hepatotoxic in humans is an IgG4 antibody that binds to CRD1. The hepatotoxicity is caused by CD8-positive T cells infiltrated into the liver.

While intensive research to overcome these problems with agonist antibodies has been conducted, there is a demand for means for activating TNFRSF signals in place of the agonist antibodies.

OX40, 4-1BB, GITR, and CD30 are not expressed in cells in a normal state, but are expressed in a case of being stimulated, and the expression is maintained for several hours to several days and then attenuated.

Therefore, in a case where genes of TNFSF ligands naturally present in viral vectors or the like are expressed to activate TNFRSF signals in place of antibodies, the ligands need to be supplied in accordance with the expression of the receptors. Since typical vectors for gene supply infect target cells and the target protein is supplied after the genes are transcribed and translated, a time lag occurs in ligand expression, and the timing of receptor expression and the timing and the amount of ligand supply are difficult to adjust.

The present invention has been made in consideration of the above-described circumstances, and an object thereof is to provide an anticancer agent, a pharmaceutical composition for cancer treatment, and a kit having excellent antitumor effects.

### [Solution to Problem]

The present invention includes the following aspects.
[1] An anticancer agent containing: a virus presenting a desired protein or peptide on a virus particle envelope, as an active ingredient.
[2] The anticancer agent according to [1], in which the virus presents at least one TNF receptor superfamily ligand on the virus particle envelope.
[3] The anticancer agent according to [2], in which the at least one TNF receptor superfamily ligand is OX40L, 4-1BBL, GITRL, CD27L, CD30L, or a variant thereof having an identical function.
[4] The anticancer agent according to [3], in which a cytoplasmic tail (CT) sequence of the OX40L, 4-1BBL, GITRL, CD27L, CD30L, or variant thereof having an identical function is replaced with a CT sequence derived from HN of the virus.
[5] The anticancer agent according to any one of [1] to [4], in which the virus is human parainfluenza virus type 2 (hPIV2).
[6] The anticancer agent according to [5], in which the human parainfluenza virus type 2 is a non-proliferative type virus in which an F gene is deleted from a genome.
[7] The anticancer agent according to [5] or [6], in which the human parainfluenza virus type 2 has an inactivated genome.
[8] A pharmaceutical composition for cancer treatment, containing: the anticancer agent according to any one of [1] to [7].
[9] The pharmaceutical composition for cancer treatment according to [8], further containing: a second anticancer agent.
[10] The pharmaceutical composition for cancer treatment according to [9], in which the second anticancer agent contains an immune activator and/or an immune checkpoint inhibitor.
[11] The pharmaceutical composition for cancer treatment according to [9] or [10], in which the second anticancer agent contains an anti-OX40 agonist antibody, an anti-4-1BB agonist antibody, an anti-GITR agonist antibody, an anti-CD27 agonist antibody, or an anti-CD30 agonist antibody.
[12] The pharmaceutical composition for cancer treatment according to any one of [8] to [11], further containing: a lipopolysaccharide (LPS) derived from Pantoea agglomerans.
[13] The pharmaceutical composition for cancer treatment according to any one of [8] to [12], in which the pharmaceutical composition is used for intratumoral administration.
[14] A kit for use in cancer treatment, which is used simultaneously or sequentially in the treatment, the kit containing: a virus presenting a desired protein or peptide on a virus particle envelope; and a second anticancer agent.
[15] The kit according to [14], in which the virus presents at least one TNF receptor superfamily ligand on the virus particle envelope.
[16] The kit according to [15], in which the at least one TNF receptor superfamily ligand is OX40L, 4-1BBL, GITRL, CD27L, CD30L, or a variant thereof having an identical function.
[17] The kit according to [16], in which a cytoplasmic tail (CT) sequence of the OX40L, 4-1BBL, GITRL, CD27L, CD30L, or variant thereof having an identical function is replaced with a CT sequence derived from HN of the virus.
[18] The kit according to any one of [14] to [17], in which the virus is human parainfluenza virus type 2.
[19] The kit according to [18], in which the human parainfluenza virus type 2 is a non-proliferative type virus in which an F gene is deleted from a genome.
[20] The kit according to [18] or [19], in which the human parainfluenza virus type 2 has an inactivated genome.
[21] The kit according to any one of [14] to [20], in which the second anticancer agent contains an immune activator and/or an immune checkpoint inhibitor.
[22] The kit according to any one of [14] to [21], in which the second anticancer agent contains an anti-OX40 agonist antibody, an anti-4-1BB agonist antibody, an anti-GITR agonist antibody, an anti-CD27 agonist antibody, or an anti-CD30 agonist antibody.
[23] The kit according to any one of [14] to [22], further containing: a lipopolysaccharide (LPS) derived from Pantoea agglomerans.
[24] The kit according to any one of [14] to [23], in which the kit is used for intratumoral administration.
[25] An anticancer agent containing: a lipopolysaccharide (LPS) derived from Pantoea agglomerans.
[26] A pharmaceutical composition for cancer treatment, containing: the anticancer agent according to [25].
[27] The pharmaceutical composition for cancer treatment according to [26], further containing: a virus presenting a desired protein or peptide on an envelope.
[28] The pharmaceutical composition for cancer treatment according to [27], in which the virus presents at least one TNFSF ligand on a virus particle envelope.
[29] The pharmaceutical composition for cancer treatment according to [28], in which the at least one TNFSF ligand is OX40L, 4-1BBL, GITRL, CD27L, CD30L, or a variant thereof having an identical function.
[30] The pharmaceutical composition for cancer treatment according to [29], in which a cytoplasmic tail (CT) sequence of the OX40L, 4-1BBL, GITRL, CD27L, CD30L, or variant thereof having an identical function is replaced with a CT sequence derived from HN of the virus.
[31] The pharmaceutical composition for cancer treatment according to any one of [27] to [30], in which the virus is human parainfluenza virus type 2.
[32] The pharmaceutical composition for cancer treatment according to [31], in which the human parainfluenza virus type 2 is a non-proliferative type virus in which an F gene is deleted from a genome.
[33] The pharmaceutical composition for cancer treatment according to [31] or [32], in which the human parainfluenza virus type 2 has an inactivated genome.
[34] The pharmaceutical composition for cancer treatment according to any one of [26] to [33], further containing a second anticancer agent.
[35] The pharmaceutical composition for cancer treatment according to [34], in which the second anticancer agent contains an immune activator and/or an immune checkpoint inhibitor.
[36] The pharmaceutical composition for cancer treatment according to [34] or [35], in which the second anticancer agent contains an anti-OX40 agonist antibody, an anti-4-1BB agonist antibody, an anti-GITR agonist antibody, an anti-CD27 agonist antibody, or an anti-CD30 agonist antibody.
[37] The pharmaceutical composition for cancer treatment according to any one of [26] to [36], in which the pharmaceutical composition is used for intratumoral administration.
[38] A kit for use in cancer treatment, which is used simultaneously or sequentially in the treatment, the kit containing: a lipopolysaccharide (LPS) derived from Pantoea agglomerans; and a virus presenting a desired protein or peptide on a virus particle envelope.
[39] The kit according to [38], in which the virus presents at least one TNFSF ligand on an envelope.
[40] The kit according to [39], in which the at least one TNFSF ligand is OX40L, 4-1BBL, GITRL, CD27L, CD30L, or a variant thereof having an identical function.
[41] The kit according to [40], in which a cytoplasmic tail (CT) sequence of the OX40L, 4-1BBL, GITRL, CD27L, CD30L, or variant thereof having an identical function is replaced with a CT sequence derived from HN of the virus.
[42] The kit according to any one of [38] to [41], in which the virus is human parainfluenza virus type 2.
[43] The kit according to [42], in which the human parainfluenza virus type 2 is a non-proliferative type virus in which an F gene is deleted from a genome.
[44] The kit according to [42] or [43], in which the human parainfluenza virus type 2 has an inactivated genome.
[45] The kit according to any one of [38] to [44], further containing: a second anticancer agent.
[46] The kit according to [45], in which the second anticancer agent contains an immune activator and/or an immune checkpoint inhibitor.
[47] The kit according to [45] or [46], in which the second anticancer agent contains an anti-OX40 agonist antibody, an anti-4-1BB agonist antibody, an anti-GITR agonist antibody, an anti-CD27 agonist antibody, or an anti-CD30 agonist antibody.
[48] The kit according to any one of [38] to [47], in which the kit is used for intratumoral administration.
[49] A pharmaceutical composition for cancer treatment, containing at least two selected from the group consisting of an OX40L or anti-OX40 agonist antibody, a 4-1BBL or anti-4-1BB agonist antibody, a GITRL or anti-GITR agonist antibody, a CD27L or anti-CD27 agonist antibody, and a CD30L or anti-CD30 agonist antibody.

### [Advantageous Effects of Invention]

According to the present invention, it is possible to provide an anticancer agent, a pharmaceutical composition for cancer treatment, and a kit having excellent antitumor effects.

### [Brief Description of Drawings]

Fig. 1 is an explanatory diagram showing a plasmid for preparing BC-PIV/OX40L, 4-1BBL, GITRL, CD27L and CD30L. Specifically, Fig. 1 is a construction diagram for introducing a desired gene into the NotI or Mlul restriction enzyme cleavage site of the plasmid for constructing hPIV2 from which the F gene has been deleted.
Fig. 2 is a diagram confirming expression and incorporation of OX40L in BC-PIV-infected cells into which mouse OX40L has been introduced and BC-PIV particles according to Western blotting.
Fig. 3 is a diagram confirming expression and incorporation of 4-1BBL in BC-PIV-infected cells into which mouse 4-1BBL has been introduced and BC-PIV particles according to Western blotting
Fig. 4 is a diagram confirming incorporation of human OX40L in BC-PIV particles into which human OX40L has been introduced according to Western blotting.
Fig. 5 is a diagram confirming expression and incorporation of human 4-1BBL in BC-PIV-infected cells into which human 4-1BBL has been introduced and BC-PIV particles according to Western blotting.
Fig. 6 is a diagram confirming incorporation of mouse GITRL and human GITRL in BC-PIV particles into which mouse GITRL and human GITRL have been introduced according to Western blotting.
Fig. 7A is a diagram confirming incorporation of mouse CD27L and human CD27L in BC-PIV particles into which mouse CD27L and human CD27L have been introduced according to Western blotting.
Fig. 7B is a diagram confirming incorporation of mouse CD30L and human CD30L in BC-PIV particles into which mouse CD30L and human CD30L have been introduced according to Western blotting.
Fig. 8 is a diagram showing a combination of two ligands, which are a mouse TNFSF ligand and a human TNFSF ligand, introduced into BC-PIV.
Fig. 9 is a diagram confirming both incorporations in BC-PIV particles, in which both mouse OX40L and mouse 4-1BBL have been introduced into BC-PIV, according to Western blotting.
Fig. 10 is a diagram confirming both incorporations in BC-PIV particles, in which both mouse GITRL and mouse OX40L or both mouse GITRL and mouse 4-1BBL have been introduced into BC-PIV, according to Western blotting.
Fig. 11 is a diagram confirming both incorporations in BC-PIV particles, in which both human OX40L and human 4-1BBL have been introduced into BC-PIV, according to Western blotting.
Fig. 12 is a diagram confirming binding of a mouse OX40 receptor or 4-1BB receptor protein to mouse OX40L or 4-1BBL-carrying BC-PIV.
Fig. 13 is a diagram confirming binding of a human OX40 receptor protein to human OX40L-carrying BC-PIV.
Fig. 14 is a diagram confirming the state of the multimeric structure of mouse 4-1BBL, 4-1BBL-PIV2-HN CT, and human CD27L incorporated into BC-PIV in a nonreduced or reduced state.
Fig. 15 is a silver staining diagram of LPS prepared by an extraction method using heat (121°C for 20 minutes) and cold EtOH from Zizania latifolia symbiotic Pantoea agglomerans.
Fig. 16 is a diagram confirming activation of NK cells due to LPS prepared by an extraction method using heat (121°C for 20 minutes) and cold EtOH.
Fig. 17 is a diagram showing temporal antitumor effects obtained by administration of BC-PIV, BC-PIV/OX40L, and the anti-OX40 agonist antibody to CT26 colorectal cancer.
Fig. 18 is a diagram showing antitumor effects of BC-PIV, the anti-OX40 agonist antibody, and LPS on CT26 colorectal cancer.
Fig. 19 is a diagram showing antitumor effects of BC-PIV, the anti-OX40 agonist antibody, and LPS on CT26 colorectal cancer.
Fig. 20 is a diagram showing the effect of LPS on CT26 colorectal cancer in terms of change in tumor volume with time.
Fig. 21 is a diagram showing the state of proliferation of CT26 tumor 4 days after administration of BC-PIV/OX40L or the like in the presence or absence of an NK cell inhibitor and a CD4/8-positive T cell inhibitor.
Fig. 22 is a diagram showing the state of proliferation of CT26 tumor 10 days after administration of BC-PIV/OX40L or the like in the presence or absence of an NK cell inhibitor and a CD4/8-positive T cell inhibitor.
Fig. 23 is a diagram showing the state of proliferation of CT26 tumor 4 days after administration of BC-PIV/4-1BBL-PIV2-HNCT or the like in the presence or absence of an NK cell inhibitor and a CD4/8-positive T cell inhibitor.
Fig. 24 is a diagram showing the state of proliferation of CT26 tumor 10 days after administration of BC-PIV/4-1BBL-PIV2-HNCT or the like in the presence or absence of an NK cell inhibitor and a CD4/8-positive T cell inhibitor.
Fig. 25 is a diagram showing temporal antitumor effects obtained by administration of BC-PIV/OX40L and the like to CT26 colorectal cancer in the presence or absence of an NK cell inhibitor and a CD4/8-positive T cell inhibitor.
Fig. 26 is a diagram showing temporal antitumor effects obtained by administration of BC-PIV/4-1BBL-PIV2-HNCT and the like to CT26 colorectal cancer in the presence or absence of an NK cell inhibitor and a CD4/8-positive T cell inhibitor.
Fig. 27 is a diagram showing temporal antitumor effects obtained by administration of BC-PIV/4-1BBL-PIV2-HNCT and the like to B16 mouse melanoma tumor of a B6 mouse.
Fig. 28 is a diagram showing the state of B16 mouse melanoma tumor of the B6 mouse 3 days after administration of BC-PIV/4-1BBL-PIV2-HN CT and the like.
Fig. 29 is a diagram showing antitumor effects of BC-PIV prepared by introducing OX40L or 4-1BBL-PIV2-HN CT genes into the NotI site of a plasmid for virus preparation.
Fig. 30 is a diagram showing antitumor effects of BC-PIV into which two genes, OX40L and 4-1BBL, have been introduced.
Fig. 31 is a diagram showing antitumor effects of two ligand protein-carrying BC-PIVs of GITRL, CD27L, or CD30L single-carrying BC-PIV and GITRL/OX40L or GITRL/4-1BBL.
Fig. 32 is a diagram showing antitumor effects of inactivated BC-PIV/OX40L or inactivated BC-PIV/4-1BBL.
Fig. 33 is a diagram showing antitumor effects of BC-PIV into which CD30L has been introduced.
Fig. 34 is a diagram showing antitumor effects of inactivated BC-PIV/OX40L or inactivated BC-PIV/4-1BBL.
Fig. 35 is a diagram showing incorporation of human OX40L into an enveloped virus (RS virus) other than hPIV2.
Fig. 36 is a diagram showing incorporation of human OX40L into an enveloped virus (PIV5) other than hPIV2.

### [Description of Embodiments]

### [OX40L, 4-1BBL, GITRL, CD27L (CD70), and CD30L (CD153)]

OX40 (CD134), 4-1BB (CD137), GITR (CD357), CD27, and CD30 which are the receptors of these ligands are members of the tumor necrosis factor receptor superfamily (TNFRSF). OX40, 4-1BB, GITR, and CD30 are inducibly expressed in activated CD4-positive T cells, activated CD8-positive T cells, NK cells, and the like.

An increase in OX40 and 4-1BB-positive lymphocytes in tumors of patients with a good prognosis has been reported (see Non Patent Document 1 and Non Patent Document 2). In activated CD4-positive T cells and CD8-positive T cells, signal transmission through binding of a TNFSF receptor and a ligand, production of inhibitory cytokines of regulatory T cells, and the like are induced, and tumor-specific CTL is induced together with TCR signals and CD28 signals for tumor antigens.

### [OX40 (CD134) and OX40L]

In a case where trimer OX40 present on the membrane of an activated CD4-positive T cell or the like binds to OX40L which is also a trimer and present on the surface of an antigen-presenting cell (APC), various functions are activated. It is considered that formation of homotrimers of OX40 and OX40L and maintenance of the three-dimensional structures are important for efficient activation, and the expression is induced by the interaction of CD40-CD40L and the stimulation of Toll-like receptors and the like.

OX40L is a molecule that is induced by dendritic cells or the like 24-120 hours after antigen stimulation (see Non Patent Document 3). It has been considered that OX40L is mainly expressed in antigen-presenting cells (APC) such as activated B cells and dendritic cells, but expression in NK cells and mast cells has also been confirmed.

The homology between the amino acid sequences of human OX40L and mouse OX40L are not so high, but the three-dimensional structures thereof are similar to each other. Due to the binding of OX40 to OX40L, activation of PI3K/PKB, NF-kB, and NFAT signals causes proliferation, survival, cytokine production, and the like of T cells.

In particular, the function from the binding of OX40 to OX40L between CD4-positive T cells and antigen-presenting cells has been intensively examined, and activated CD4-positive T cells secrete various cytokines. It is known that Th2 type T cells are differentiated by the autocrine action due to IL-4 secretion and differentiated into Th1 type T cells due to the secretion of IL-12 and Th1 type cytokines.

In particular, Th1 type T cells contribute to the activation of CTL that directly attacks cancer cells. Further, it is known that OX40 is constitutively expressed in regulatory T cells that negatively control immunity and the expression of OX40L of APC suppresses the action of the regulatory T cells, and the OX40/OX40L signals suppress the negative immune system and contribute to the antitumor effects.

NK cells are made from progenitor cells in the bone marrow and target cancer cells, virus-infected cells, inflammatory cells to which ultraviolet rays and oxidative stress have been applied, and the like. The NK cells are capable of exhibiting cytotoxic activity without presensitization with an antigen. Although it has been reported that OX40 is expressed in NK cells, there have been few reports on the functions thereof.

However, it has recently been reported that the survival time of mice is extended by 40% by adding an anti-OX40 agonist antibody in addition to the anti-CD20 antibody in treatment models using mouse B cell malignant lymphoma cell line (BCL₁) (See Non Patent Document 4). Since extension of the survival time was not recognized in a case where NK cells are suppressed with an asialo-GMl antibody, the expression of OX40 in NK cells and the signals thereof are considered to contribute to the antitumor effects.

It has been reported that in an in vitro test of human NK cells, the expression of 4-1BB is also increased on NK cells in addition to OX40, for example, the expression of OX40 is significantly increased after 24 hours and the expression of 4-1BB is significantly increased after 4 hours from the anti-CD20 antibody stimulation (see Non Patent Document 4). This indicates that the expression of OX40L and 4-1BBL that bind to the OX40 receptor and the 4-1BB receptor also needs to occur early after stimulation. Further, this also indicates that contact with T cells and monocyte cells is required for the expression of OX40 on NK cells, and it is considered that even though NK cells exhibit cytotoxic activity without presensitization with an antigen, cooperation with T cells is important for exhibiting the antitumor effects, and efficient antitumor immunity is not induced simply by NK cells alone.

### [4-1BB and 4-1BBL]

Similar to OX40 and OX40L, 4-1BB and 4-1BBL are also trimers and have enhanced functions (see Non Patent Documents 17 and 18), and the functions thereof are similar to the functions of OX40 and OX40L in CD4-positive T cells and NK cells. Meanwhile, the expression of OX40 is strongly suppressed by thalidomide-treated Treg cells, but it is considered that 4-1BB is not changed (see Non Patent Document 5) and the functions of OX40 and 4-1BB are not exactly the same as each other.

### [Anti-OX40 agonist antibody and anti-4-1BB agonist antibody]

Almost no antitumor effect is exhibited by administration of recombinant OX40L and 4-1BBL monomeric protein, and anti-OX40 agonist antibodies or anti-4-1BB agonist antibodies are typically used to activate TNFRSF signals. The tumor regression and tumor proliferation effects obtained by using these agonist antibodies in non-clinical tumor treatment tests have reported multiple times.

Further, clinical tests using anti-OX40 agonist antibodies and anti-4-1BB agonist antibodies have been performed multiple times (see Non Patent Documents 6, 7, and 8).

In addition, antitumor effects of the anti-4-1BB agonist antibody Urelumab are exhibited in the clinical tests, but hepatotoxicity is reported (see Non Patent Document 15). Recently, the properties of Urelumab and Utomilumab, which is another anti-4-1BB agonist antibody, have been investigated (see Non Patent Document 16). The 4-1BB receptor has four cysteine-rich domains (CRD1-4), and the signals are activated by binding ligands or agonist antibodies to these domains. Urelumab is a human IgG4 antibody that binds to CRD1 and Utomilumab is a human IgG2 antibody that binds to CRD3 and CRD4, and the antibody properties therebetween are different from each other. In co-culture tests of these antibodies with mouse spleen cells and CD8-positive T cells, Urelumab can induce IPNγ in co-culture with any of the spleen cells and the CD8-positive T cells, whereas Utomilumab can induce IPNγ in co-culture with the spleen cells, but cannot induce IPNγ in co-culture with only the CD8-positive T cells. Based on the results, it is considered that the CD8-positive T cells activated by Urelumab are activated independently, and the CD8-positive T cells are infiltrated into the liver and thus the hepatotoxicity is induced.

In regard to the agonist antibodies, it is considered that there are still problems to be solved in terms of safety, such as the binding site with CRD and the binding with Fc-FcyR.

### [GITR and GITRL]

GITR (glucocorticoid-induced tumor necrosis factor receptor) is most abundantly expressed in activated CD4/8-positive T cells and regulatory T cells similarly to OX40 and 4-1BB. The binding of GITR to GITRL activates TNFRSF signals and also contributes to the activation of the T cell receptor (TCR) and the suppression of activity of the regulatory T cells. The expression is increased 24 to 72 hours after the stimulation and continues for several days. In a mouse antitumor test carried out using a GITR agonist antibody, the GITR agonist antibody reduces regulatory T cells in the tumor and increases CTL activity. Development of a large number of anti-GITR agonist antibodies has been promoted.

### [CD27 and CD27L]

CD27 is a receptor molecule belonging to TNFRSF, and CD27L (CD70) is a ligand that serves as a counterpart thereof. CD27L is expressed in malignant tumor cells such as diffuse large B-cell lymphoma and follicular lymphoma. Receptor CD27 is considered to be constitutively expressed in naive T cells and regulatory T cells, and the expression state thereof is different from those of OX40, 4-1BB, GITR, and CD30. Due to the binding of CD27 to CD27L, memory functions and effector functions such as assistance for T cell activation and differentiation of T cells are exerted. The phase 2 test is performed on agonist antibodies against CD27 by Celldex for ovarian cancer, head and neck cancer, glioblastoma, and renal cell cancer. An increase of CD27L in synovial-derived T cells and the like in patients with rheumatoid arthritis and patients with psoriatic arthritis has been recognized.

### [CD30 and CD30L]

CD30 is a receptor molecule belonging to TNFRSF, and CD30L is a ligand that serves as a counterpart thereof and is expressed in activated CD4/8-positive T cells. CD30 is associated with various lymphomas containing anaplastic large cells.

In the recent document, it has been reported that unmethylated CpG DNA that activates Toll-like receptors (TLRs) 9 can increase the expression of OX40 in CD4-positive T cells, and in a case where an anti-OX40 agonist antibody together with CpG are administered to one site of multiple transplanted solid cancers, the effect of regressing cancer not only at the administration site but also at a distant site (see Non Patent Document 9). That is, only administration of agonist antibodies of anti-OX40, anti-4-1BB, anti-GITR, anti-CD27, and anti-CD30 is not sufficient for administration to solid cancers, regression of distant cancers, and suppression of proliferation of cancers, and expression of these receptors CD4-positive T cells and NK cells is important.

### [BC-PIV vector and VLP. BC-PIV vector]

The present inventors constructed a BC-PIV vector prepared by allowing the F gene encoding the membrane fusion protein (F membrane protein or F protein) of human parainfluenza virus type 2 (hereinafter, referred to as hPIV2) belonging to the family Paramyxoviridae, subfamily Paramyxoviridae, genus Rubulavirinae, which is a negative single-stranded RNA virus, to be deleted so that the F gene does not function, establishing Vero cells integrated such that the F gene is constitutively expressed, and preventing production of secondary infectious particles in the cells (see Non Patent Document 10).

In the vector, high safety is ensured because secondary infectious particles are not produced in the infected host. Foreign genes can be introduced into the vector, and high expression of foreign genes in infected cells is recognized by utilizing the properties of RNA vectors.

In addition, BC-PIV is characterized by antigen introduction. According to the characteristic, BC-PIV can coexist not only with antigen genes but also with HN membrane protein and F membrane protein of hPIV2 on the BC-PIV envelope in a state where the three-dimensional structure of the large antigen protein such as the outer membrane of the foreign virus is maintained, and thus BC-PIV can be used as an efficient vaccine vector (see Non Patent Document 10).

Induction of a neutralizing antibody is succeeded by introducing a high-molecular-weight membrane protein of a virus other than the virus, which requires a three-dimensional structure for inducing a neutralizing antibody, into the vector. In addition, BC-PIV in which an antigen is introduced onto the envelope can be used as a vector even in a case where the genome is inactivated (VLP. BC-PIV). In a case of inactivation, since BC-PIV is a non-proliferative vector, the treatment can be conducted with a drug having a significantly lower concentration than that of a typical virus inactivation treatment, and the immunogenicity of the introduced antigen can be maintained (see Non Patent Document 11 and Patent Document 2).

In a preferred embodiment, BC-PIV provides a therapeutic agent for treating solid cancer, and the therapeutic agent carries OX40L-PIV2-HN CT genes in which the cytoplasmic tail (CT) sequence of the intracellular region of an OX40L gene or OX40L is replaced with the CT sequence of hPIV2 HN

In the embodiment, a method of injecting the vector directly into solid cancer is also provided. OX40L or OX40L-PIV2-HN CT is incorporated onto BC-PIV or VLP. BC-PIV not only in the genetic form but also in the protein form. The protein interacts with OX 40 receptors of T cells, NK cells, or the like activated in a short time due to priming with BC-PIV or the like to cause the immune effects against tumors in combination with the cytotoxic activity due to BC-PIV recognition of NK cells and production effects of cytokines such as IFNγ.

One preferred form is an enveloped virus that includes OX40L having an extramembrane region on the virus envelope. A more preferred form is VLP. BC-PIV that carries BC-PIV or OX40L or OX40L-PIV2-HNCT in the protein form which carries OX40L or OX40L-PIV2-HN CT in the genetic and the protein form.

In another preferred embodiment, BC-PIV provides a therapeutic agent for treating solid cancer, and the therapeutic agent carries a 4-1BBL-PIV2-HN CT gene in which the cytoplasmic tail (CT) sequence of the intracellular region of the 4-1BBL gene or 4-1BBL is replaced with the CT sequence of hPIV2 HN

In the embodiment, a method of injecting the vector directly into solid cancer is also provided. 4-1BBL or 4-1BBL-PIV2-HN CT is incorporated onto BC-PIV or VLP. BC-PIV not only in the genetic form but also in the protein form. The protein interacts with 4-1BB receptors of T cells, NK cells, or the like activated in a short time due to priming with BC-PIV or the like to cause the immune effects against tumors in combination with the cytotoxic activity due to BC-PIV recognition of NK cells and production effects of cytokines such as IFNγ.

One preferred form is an enveloped virus that includes 4-1BBL having an extramembrane region on the virus envelope. A more preferred form is VLP. BC-PIV that carries BC-PIV or 4-1BBL or 4-1BBL-PIV2-HNCT in the protein form which carries 4-1BBL or 4-1BBL-PIV2-HN CT in the genetic and the protein form.

In another preferred embodiment, BC-PIV provides a therapeutic agent for treating solid cancer, and the therapeutic agent carries a GITRL-PIV2-HN CT gene in which the cytoplasmic tail (CT) sequence of the intracellular region of the GITRL gene or GITRL is replaced with the CT sequence of hPIV2 HN

In the embodiment, a method of injecting the vector directly into solid cancer is also provided. GITRL or GITRL-PIV2-HN CT is incorporated onto BC-PIV or VLP. BC-PIV not only in the genetic form but also in the protein form. The protein interacts with GITR receptors of T cells, NK cells, or the like activated in a short time due to priming with BC-PIV or the like to cause the immune effects against tumors in combination with the cytotoxic activity due to BC-PIV recognition of NK cells and production effects of cytokines such as IFNγ.

One preferred form is an enveloped virus that includes GITRL having an extramembrane region on the virus envelope. A more preferred form is VLP. BC-PIV that carries BC-PIV or GITRL or GITRL-PIV2-HN CT in the protein form which carries GITRL or GITRL-PIV2-HN CT in the genetic and the protein form.

In another preferred embodiment, BC-PIV provides a therapeutic agent for treating solid cancer, and the therapeutic agent carries a CD27L-PIV2-HN CT gene in which the cytoplasmic tail (CT) sequence of the intracellular region of the CD27L gene or CD27L is replaced with the CT sequence of hPIV2 HN

In the embodiment, a method of injecting the vector directly into solid cancer is also provided. CD27L or CD27L-PIV2-HN CT is incorporated onto BC-PIV or VLP. BC-PIV not only in the genetic form but also in the protein form. The protein interacts with CD27 receptors of T cells, NK cells, or the like activated in a short time due to priming with BC-PIV or the like to cause the immune effects against tumors in combination with the cytotoxic activity due to BC-PIV recognition of NK cells and production effects of cytokines such as IFNγ.

One preferred form is an enveloped virus that includes CD27L having an extramembrane region on the virus envelope. A more preferred form is VLP. BC-PIV that carries BC-PIV or CD27L or CD27L-PIV2-HN CT in the protein form which carries CD27L or CD27L-PIV2-HN CT in the genetic and the protein form.

In another preferred embodiment, BC-PIV provides a therapeutic agent for treating solid cancer, and the therapeutic agent carries a GITR-PIV2-HN CT gene in which the cytoplasmic tail (CT) sequence of the intracellular region of the CD30L gene or CD30L is replaced with the CT sequence of hPIV2 HN

In the embodiment, a method of injecting the vector directly into solid cancer is also provided. CD30L or CD30L-PIV2-HN CT is incorporated onto BC-PIV or VLP. BC-PIV not only in the genetic form but also in the protein form. The protein interacts with CD30 receptors of T cells, NK cells, or the like activated in a short time due to priming with BC-PIV or the like to cause the immune effects against tumors in combination with the cytotoxic activity due to BC-PIV recognition of NK cells and production effects of cytokines such as IFNγ.

One preferred form is an enveloped virus that includes CD30L having an extramembrane region on the virus envelope. A more preferred form is VLP. BC-PIV that carries BC-PIV or CD30L or CD30L-PIV2-HN CT in the protein form which carries CD30L or CD30L-PIV2-HN CT in the genetic and the protein form.

### [BC-PIV vector and VLP. BC-PIV vector carrying multiple genes]

In a preferred embodiment, the BC-PIV vector or VLP. BC-PIV vector carries multiple genes in one vector. By adjusting the site where a foreign gene can be introduced into a plasmid for virus preparation, a viral vector that expresses two or more genes can be constructed.

As the gene to be introduced, a gene encoding a ligand of TNFSF is preferable, and two or more selected from OX40L, 4-1BBL, GITRL, TNFα, LTα, TL1A, CD30L, and CD27L, and a variant thereof having the identical function are more preferable.

Among these, as the combination of the ligands of TNFSF, a combination of OX40L and 4-1BBL, a combination of GITRL and OX40L, or a combination of GITRL and 4-1BBL is more preferable.

To present a combination of these ligands on the envelope, it is preferable that the cytoplasmic tail (CT) sequence of OX40L, 4-1BBL, GITRL, CD27L, CD30L, or variant thereof having the identical function is replaced with the CT sequence derived from HN of the virus.

### [Activation of dendritic cells (DC), NK cells, and the like due to LPS derived from Pantoea agglomerans]

For efficient induction of antitumor immunity, in addition to activation of OX40 and 4-1BB pathways such as NK cells and CD4-positive T cells, presentation of CTL epitopes of cancer cells by activated dendritic cells and the like is important.

Cancer cell-derived proteins incorporated into professional antigen-presenting cells such as dendritic cells by the action of endocytosis or the like are cleaved into various lengths of peptides due to proteases. Dendritic cells present degraded 8 to 10 amino acid-length CTL epitope peptides derived from incorporated cancer cells onto major histocompatibility complex (MHC) class I molecules on the cell surface by cross-presentation, and peptide information is transmitted to CD8-positive T cells, which leads to CTL activation.

In addition, in an immature state in which co-stimulatory molecules such as CD80, CD86, and CD40 are not induced, dendritic cells only give stimulation through an antigen signal (first signal) of MHC molecules during naive T cell stimulation, and the co-stimulatory signal (second signal) does not enter, and finally T cells enter a state (anergy) in which the T cells cannot respond to the antigen thereof. Dendritic cells need to transmit and mature the second signal due to the expression of co-stimulatory molecules such as CD80, CD86, and CD40 in order to prevent the cells from entering an anergy state with respect to tumor antigens.

The present inventors previously isolated Pantoea agglomerans which is a Gram-negative bacterium from the enlarged flesh of Zizania latifolia (also known as Manchurian Wild Rice) which is perennial herb belonging to the genus Zizania latifolia of the Gramineae family in a sterilized safety cabinet. It has been suggested that lipopolysaccharides (LPS) extracted from the bacteria induce the expression of co-stimulatory molecules such as CD80, CD86, and CD40 in immature dendritic cells and strongly mature the dendritic cells (see Patent Document 3). Unlike LPS such as Escherichia coli, LPS derived from Pantoea agglomerans is considered to have extremely low toxicity in animal tests.

In addition, activation of NK cells indirectly or directly due to LPS has been reported (see Non Patent Document 12). In terms of indirect activation, dendritic cells and macrophages are activated through TLR4, and thus NK cells are activated. In terms of direct activation, the expression of TLR4 is usually considered to be low in NK cells, but it has been reported that LPS stimulation reduces the degradation of NK cells as the production of IFNγ from NK cells increases.

In a preferred embodiment, a method of treating a solid cancer is provided, and the method includes injecting LPS of Pantoea agglomerans directly into the solid cancer. By the action of the LPS, killing of cancer cells by the innate immune effect through NK cell activation in the cancer tissues, presentation of the tumor antigen epitope to the CD4-positive T cells through the dendritic cell activation, and killing of cancer cells by the acquired immunity through CTL activation are attempted.

According to a still another preferred embodiment, a method of mixing a vector containing BC-PIV or VLP. BC-PIV, OX40L, 4-1BBL, GITRL, CD27L or CD30L, OX40L, 4-1BBL, GITRL, CD27L or CD30L-PIV2-HN CT proteins in addition to OX40L, 4-1BBL, GITRL, CD27L or CD30L genes in an envelope with LPS of Pantoea agglomerans and injecting the mixture directly into a solid cancer is provided.

### <Anticancer agent>

### [First embodiment]

In one embodiment, the present invention provides an anticancer agent containing a virus capable of presenting a desired protein or peptide on a virus particle envelope, as an active ingredient.

As described below in the examples, antitumor effects are obtained even in a case of a so-called empty viral vector into which a gene encoding a desired protein or peptide has not been introduced.

The virus used in the present embodiment is not limited as long as the virus is capable of presenting a desired protein or peptide on the envelope, and examples thereof include Sendai virus and human parainfluenza virus. Among these, human parainfluenza virus is preferable, and human parainfluenza virus type 2 is more preferable.

As described above, from the viewpoint that secondary infectious particles are not produced, it is preferable that human parainfluenza virus type 2 (hPIV2) lacks the F gene having a membrane fusion function and is non-proliferative.

Further, from the viewpoint of the safety, it is preferable that the genome of human parainfluenza virus type 2 is inactivated. Genome inactivation is carried out preferably by an alkylating agent treatment, a hydrogen peroxide treatment, UV irradiation, radiation irradiation, or a heat treatment and more preferably by treatment with β-propiolactone which is an alkylating agent.

In addition, it is preferable that the virus used in the present embodiment presents at least one TNFSF ligand on the envelope. As at least one TNFSF ligand, at least one selected from OX40L, 4-1BBL, GITRL, TNFα, LTα, TL1A, CD30L, and CD27L is preferable, and at least one selected from the group consisting of OX40L, 4-1BBL, GITRL, CD27L, CD30L, and a variants thereof having the identical function is more preferable.

These variants having the identical function are formed of an amino acid sequence in which one or several amino acids are deleted, replaced, inserted, or added in the amino acid sequence constituting the ligand, and have the signal transmission ability of the TNFRSF pathway.

The number of amino acids to be deleted, replaced, inserted, or added is preferably in a range of 1 to 20, more preferably in a range of 1 to 10, and particularly preferably in a range of 1 to 5.

Further, the TNFSF ligand used in this embodiment also include a homologue thereof.

To present a desired protein or peptide on the envelope, it is preferable that the cytoplasmic tail (CT) sequence of OX40L, 4-1BBL, GITRL, CD27L, and/or CD30L, or variant thereof having the identical function is replaced with the CT sequence derived from HN of the virus.

### [Second embodiment]

In one embodiment, the present invention provides an anticancer agent containing a lipopolysaccharide (LPS) derived from Pantoea agglomerans as an active ingredient.

As described below in the examples, antitumor effects are obtained even in a case of using only the lipopolysaccharide (LPS) derived from Pantoea agglomerans.

### <Pharmaceutical composition for cancer treatment>

### [Third embodiment]

In one embodiment, the present invention provides a pharmaceutical composition containing the anticancer agent of the first embodiment described above. The pharmaceutical composition for cancer treatment according to the present embodiment may contain a second anticancer agent.

Examples of the second anticancer agent containing the pharmaceutical composition for cancer treatment according to the present embodiment include a cytotoxic agent, a cell proliferation quiescent agent, an immune activator, and an immune checkpoint inhibitor, and preferred examples thereof include those containing an immune activator and/or an immune checkpoint inhibitor.

Examples of the immune activator include an agonist antibody of a TNFSF receptor, and specific examples thereof include an anti-OX40 agonist antibody, an anti-GITR agonist antibody, an anti-CD30 agonist antibody, an anti-4-1BB agonist antibody, and an anti-CD27 agonist antibody.

Examples of the immune checkpoint inhibitor include an anti-PD-1 antagonist antibody, an anti-PD-Ll antagonist antibody, and an anti-CTLA-4 antagonist antibody.

In addition, preferred examples of the anticancer agent containing the pharmaceutical composition for cancer treatment according to the present embodiment include those containing an anti-OX40 agonist antibody, an anti-4-1BB agonist antibody, an anti-GITR agonist antibody, an anti-CD30 agonist antibody, and/or an anti-CD27 agonist antibody.

It is preferable that the pharmaceutical composition for cancer treatment according to the present embodiment further contains a lipopolysaccharide (LPS) derived from Pantoea agglomerans. As described below in the examples, in a case where the pharmaceutical composition contains LPS derived from Pantoea agglomerans, the antitumor effects are expected to increase.

It is preferable that the pharmaceutical composition for cancer treatment according to the present embodiment contains a pharmaceutically acceptable carrier. As the pharmaceutically acceptable carrier, those typically used in preparations can be used without particular limitation, and examples thereof include a solvent such as sterile water or physiological saline; a binder such as gelatin, corn starch, gum tragacanth, or gum arabic, and an excipient such as crystalline cellulose; and a swelling agent such as alginic acid.

The pharmaceutical composition for cancer treatment according to the present embodiment may contain an additive. Examples of the additive include a lubricant such as magnesium stearate; a sweetener such as sucrose, lactose, or saccharin; a flavoring agent such as peppermint or akamono oil; a stabilizer such as benzyl alcohol or phenol; a buffering agent such as a phosphate or sodium acetate; a solubilizing agent such as benzyl benzoate or benzyl alcohol; an antioxidant; a preservative; a surfactant; and an emulsifier.

The pharmaceutical composition for cancer treatment according to the present embodiment can be formulated by appropriately combining the above-described pharmaceutically acceptable carriers and additives and mixing those in a typically accepted unit dose form.

The pharmaceutical composition for cancer treatment according to the present embodiment may be formulated into a dosage form used orally or a dosage form used parenterally, and it is preferable that the pharmaceutical composition is formulated into the dosage form used parenterally. Examples of the dosage form used orally include a tablet, a capsule, an elixir, and a microcapsule. Examples of the dosage form used parenterally include an injection, an ointment, and a patch. Among these, an injection is preferable.

Examples of the solvent for an injection include isotonic solutions containing adjuvants such as physiological saline, glucose, D-sorbitol, D-mannose, D-mannitol, and sodium chloride. The solvent for an injection may contain alcohol such as ethanol; polyalcohol such as propylene glycol or polyethylene glycol; and a nonionic surfactant such as Polysorbate 80 (registered trademark) or HCO-50.

Examples of the administration to patients includes an intranasal method, a transbronchial method, an intramuscular method, a percutaneous method, and an oral method, in addition to intraarterial injection, intravenous injection, and subcutaneous injection, but intratumoral administration is preferable.

In a case of parental administration, a single dose of the pharmaceutical composition for cancer treatment according to the present embodiment varies depending on the administration target, the target organ, the symptoms, and the administration method. For example, in a case of a normal adult (a body weight of 60 kg) in an injection form, it is considered that administration is made by intravenous injection or local administration of an active ingredient in an amount of approximately 0.01 to 300 mg, approximately 0.1 to 200 mg, or approximately 0.1 to 100 mg per day. Further, the above-described amount of the active ingredient may be administered once or in several divided doses per day.

The application target of the pharmaceutical composition for cancer treatment according to the present embodiment is not limited as long as the target is cancer, and examples thereof include breast cancer (such as infiltrating ductal carcinoma, ductal carcinoma in situ, or inflammatory breast cancer), prostate cancer (such as hormone-dependent prostate cancer or hormone-independent prostate cancer), pancreatic cancer (such as pancreatic duct cancer), gastric cancer (such as papillary adenocarcinoma, mucinous adenocarcinoma, or adenosquamous carcinoma), lung cancer (such as non-small cell lung cancer, small cell lung cancer, or malignant mesothelioma), colon cancer (such as gastrointestinal stromal tumor), rectal cancer (such as gastrointestinal stromal tumor), colorectal cancer (such as familial colorectal cancer, hereditary nonpolyposis colorectal cancer, or gastrointestinal stromal tumor), small intestine cancer (such as non-Hodgkin lymphoma or gastrointestinal stromal tumor), esophageal cancer, duodenal cancer, tongue cancer, pharyngeal cancer (such as nasopharyngeal cancer, oropharyngeal cancer, or hypopharyngeal cancer), head and neck cancer, salivary grand cancer, brain tumors (such as pineal astrocytoma, pilocytic astrocytoma, diffuse astrocytoma, or anaplastic astrocytoma), neurinoma, liver cancer (such as primary liver cancer, extrahepatic bile duct cancer), kidney cancer (such as renal cell cancer, transitional cell carcinoma of the renal pelvis and ureter), gallbladder cancer, bile duct cancer, endometrial cancer, cervical cancer, uterine sarcoma, ovarian cancer (such as epithelial ovarian cancer, extragonal germ cell tumor, ovarian embryonic cell tumor, or low-grade ovarian tumor), bladder cancer, urethral cancer, skin cancer (such as Merkel cell cancer), hemangioma, malignant lymphoma (such as reticular sarcoma, lymphosarcoma, or Hodgkin's disease), melanoma (malignant melanoma), thyroid cancer (such as thyroid medullary carcinoma), parathyroid cancer, nasal cavity cancer, sinus cancer, bone tumors (such as osteosarcoma, Ewing's sarcoma, or soft tissue sarcoma), metastatic medulloblastoma, hemangiofibroma, dermatofibrosarcoma protuberans, retinal sarcoma, penile cancer, testicular tumor, pediatric solid cancer (such as Wilms tumor or pediatric renal tumor), Kaposi's sarcoma, Kaposi's sarcoma caused by AIDS, maxillary sinus tumors, fibrous histiocytoma, leiomyosarcoma, rhabdomyoblastoma, chronic myeloproliferative disease, and leukemia (such as acute myeloid leukemia or acute lymphocytic leukemia).

### [Fourth embodiment]

In one embodiment, the present invention provides a pharmaceutical composition for cancer treatment containing the anticancer agent according to the second embodiment described above. As described below in the examples, the antitumor effects have been confirmed even in a case of administering only LPS derived from Pantoea agglomerans.

It is preferable that the pharmaceutical composition for cancer treatment according to the present embodiment contains a virus capable of presenting a desired protein or peptide on the envelope.

In addition, it is preferable that the pharmaceutical composition contains, as the virus, a virus obtained by presenting at least one TNFSF ligand on the envelope.

The pharmaceutical composition for cancer treatment according to the present embodiment further contains preferably a second anticancer agent and more preferably, as the second anticancer agent, an immune activator and/or an immune checkpoint inhibitor.

In addition, preferred forms of the pharmaceutical composition for cancer treatment according to the present embodiment are the same as those of the third embodiment.

### [Fifth embodiment]

In one embodiment, the present invention provides a pharmaceutical composition for cancer treatment, containing at least two selected from the group consisting of an OX40L or anti-OX40 agonist antibody, a 4-1BBL or anti-4-1BB agonist antibody, a GITRL or anti-GITR agonist antibody, a CD27L or anti-CD27 agonist antibody, and a CD30L or anti-CD30 agonist antibody. As described below in the examples, synergistic antitumor effects have been confirmed by administration of a BC-PIV vector carrying a gene encoding a plurality of TNFSF ligands.

At least two molecules are selected from the above-described molecules as the combination of ligands or agonist antibodies in the pharmaceutical composition for cancer treatment according to the present embodiment. Among these, a combination of an OX40L or anti-OX40 agonist antibody and a 4-1BBL or anti-4-1BB agonist antibody, a combination of a GITRL or anti-GITR agonist antibody and an OX40L or anti-OX40 agonist antibody, or a combination of a GITRL or anti-GITR agonist antibody and a 4-1BBL or anti-4-1BB agonist antibody is preferable.

### <Kit>

In one embodiment, the present invention provides a kit for use in cancer treatment, which is used simultaneously or sequentially in the treatment, the kit containing a virus capable of presenting a desired protein or peptide on an envelope, and a second anticancer agent.

In addition, preferred forms of the kit according to the present embodiment are the same as those of the [third embodiment] in the section of the <pharmaceutical composition for cancer treatment>. The order in a case of the sequential administration is not particularly limited.

Further, in one embodiment, the present invention provides a kit for use in cancer treatment, which is used simultaneously or sequentially in the treatment, a lipopolysaccharide (LPS) derived from Pantoea agglomerans; and a virus capable of presenting a desired protein or peptide on a virus particle envelope.

In addition, preferred forms of the kit according to the present embodiment are the same as those of the [fourth embodiment] in the section of the <pharmaceutical composition for cancer treatment>. The order in a case of the sequential administration is not particularly limited.

### <Treatment method>

In one embodiment, the present invention provides a method of treating cancer, including administering the anticancer drug or pharmaceutical composition for cancer treatment described above to a patient in need of treatment.

Further, in one embodiment, the present invention provides a method of treating cancer, including simultaneously or sequentially administering a virus capable of presenting an effective amount of a desired protein or peptide on an envelope and an effective amount of a second anticancer agent to a patient in need of treatment. In addition, preferred embodiments of the treatment method according to the present embodiment are the same as those of the [third embodiment] in the section of the <pharmaceutical composition for cancer treatment>. The order in a case of the sequential administration is not particularly limited.

Further, in one embodiment, the present invention provides a method of treating cancer, including simultaneously or sequentially administering an effective amount of the lipopolysaccharide (LPS) derived from Pantoea agglomerans and a virus capable of presenting an effective amount of a desired protein or peptide on the envelope to a patient in need of treatment. In addition, preferred forms of the treatment method according to the present embodiment are the same as those of the [fourth embodiment] in the section of the <pharmaceutical composition for cancer treatment>. The order in a case of the sequential administration is not particularly limited.

### [Other embodiments]

In one embodiment, the present invention provides an anticancer agent, a pharmaceutical composition for cancer treatment, or a kit for cancer treatment. As the anticancer agent, the pharmaceutical composition for cancer treatment, and the kit, the same ones as described above can be used.

### [Examples]

Hereinafter, the present invention will be described in more detail based on the examples, but the present invention is not limited to the following examples.

In the notation of OX40L, 4-1BBL, GITRL, CD27L, CD30L, and the CT substitutes thereof in the following examples, these are assumed to be derived from a mouse in a case where it is not mentioned or described whether these are derived from animal species.

### [Experimental Example 1]

### (Construction of BC-PIV carrying each of OX40L, 4-1BBL, GITRL, CD27L, and CD30L of mouse or human)

BC-PIV can be mounted on an envelope in a state where the three-dimensional structure of an antigen of a multimer of a heterogeneous virus is maintained. In a case where the antigen is other than a membrane protein such as a foreign virus, the antigen is incorporated into the BC-PIV envelope by adding the membrane region (transmembrane domain (TM) sequence) and/or intracellular region (cytoplasmic tail (CT) sequence) of HN or F of the hPIV2 membrane protein.

Further, in a case where the antigen introduced into BC-PIV is a viral membrane protein or a membrane protein, the membrane anchoring signal of the introduced antigen is incorporated into the BC-PIV envelope as it is or the membrane anchoring signal is not incorporated into the BC-PIV envelope unless the membrane anchoring signal is replaced with that of hPIV2.

Mouse and human OX40L, 4-1BBL, GITRL, CD27L, and CD30L are proteins that carry a membrane region (transmembrane domain (TM) sequence) of the membrane anchoring and an intracellular region (cytoplasmic tail (CT) sequence), and as membrane anchoring signals, BC-PIV carrying mouse and human OX40L, 4-1BBL, GITRL, CD27L, and CD30L genes having intact sequences of mouse and human OX40L, 4-1BBL, GITRL, CD27L, and CD30L, and BC-PIV carrying mouse and human OX40L, 4-1BBL, GITRL, CD27L, and CD30L genes having sequences replaced with the CT sequences of the HN protein of hPIV2 are constructed, and incorporation into the envelope of BC-PIV was investigated.

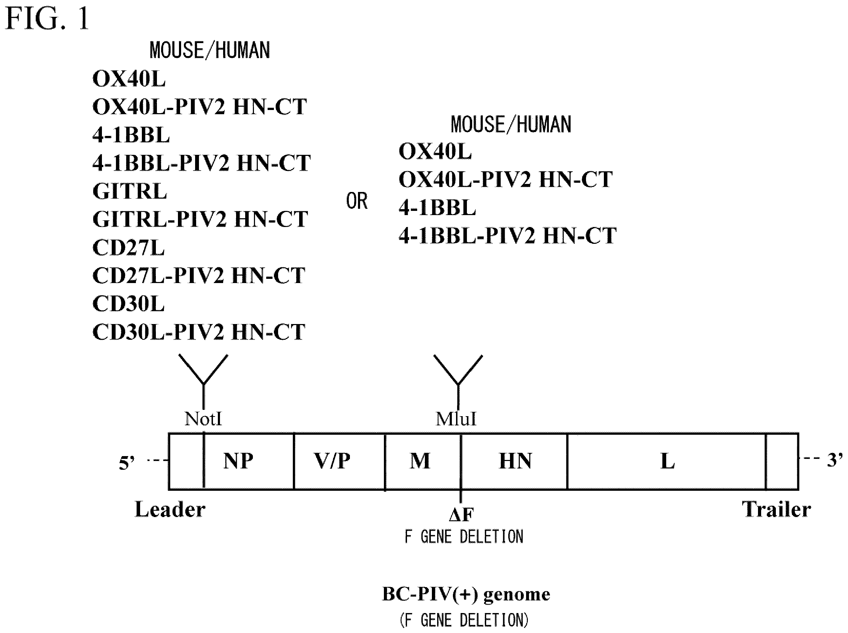

In the former case, a plasmid obtained by adding the restriction enzyme cleavage sequence and the tag sequence for BC-PIV introduction to the restriction enzyme cleavage site of Notl or Mlul of the constructed plasmid of BC-PIV was constructed with the sequences of the mouse and human OX40L, 4-1BBL, GITRL, CD27L, and CD30L genes as they were (intact) (see Fig. 1).

Here, the number of bases in the total length of genes of BC-PIV including transgenes was adjusted to be a multiple of 6 (rule of 6).

Meanwhile, in the latter case where the incorporation into the BC-PIV vector was not able to be made with the intact sequences of the mouse or human OX40L, 4-1BBL, GITRL, CD27L, and CD30L, the CT sequences of mouse or human OX40L, 4-1BBL, GITRL, CD27L, and CD30L need to be replaced with the sequence of the membrane protein of hPIV2. Since the TNFSF ligand protein is a type II membrane protein, introduction of the TM sequence and CT sequence of the F protein of hPIV2, which is a typically used type I membrane protein, is considered to be difficult.

Therefore, it is considered to use the CT sequence and the TM sequence on the N terminal side of the HN membrane protein of hPIV2, which is a type II membrane protein. Here, the HN membrane protein of hPIV2 is a tetrameric protein, and the TNFSF ligand protein is a trimer in a case where the TM sequence is replaced with the TM sequence of the TNFSF ligand protein, and there is a possibility of forming a tetramer. Therefore, a plasmid to which a tag sequence for BC-PIV introduction was added was constructed by determining introduction of the mouse or human OX40L gene (OX40L-PIV2-HN CT gene), 4-1BBL gene (4-1BBL-PIV2-HN CT gene), GITRL gene (GITRL-PIV2-HN CT gene), CD27L gene (CD27L-PIV2-HN CT gene), and CD30L gene (CD30L-PIV2-HN CT gene) in which the CT sequences of mouse or human OX40L, 4-1BBL, GITRL, CD27L, and CD30L were replaced with only the CT sequence (N-terminal-MEDYSNLSLK SIPKRTCRII FRTAT) of the HN membrane protein of hPIV2 (see Fig. 1).

The BC-PIV virus was recovered using the plasmid for constructing BC-PIV which carries these genes according to the reverse genetics method commonly used for preparing a negative single-stranded virus. All the viruses were able to be recovered.

The mouse OX40L gene sequence used here was GenBank: U12763.1 (SEQ ID NO: 1), and the human OX40L gene sequence was GenBank: NM_003326.3 (Sino Biological Inc.) (SEQ ID NO: 2).

The mouse 4-1BBL gene sequence used here was GenBank: L15435.1 (SEQ ID NO: 3), and the human 4-1BBL gene sequence was GenBank: NM_003811.3 (Sino Biological Inc.) (SEQ ID NO: 4).

The mouse GITRL gene sequence used here was GenBank: NM_183391.3 (SEQ ID NO: 5), and the human GITRL gene sequence was GenBank: NM_005092.3 (Sino Biological Inc.) (SEQ ID NO: 6).

The mouse CD27L gene used here was recovered from the mouse spleen by PCR. The gene sequence was GenBank: U78091.1 (SEQ ID NO: 7), and the human CD27L gene was recovered from MT2 cells by PCR. The sequence was GenBank: BC000725.2 (SEQ ID NO: 8).

The mouse CD30L gene used here was recovered from the mouse spleen by PCR. The gene sequence was GenBank: BC117097.1. (SEQ ID NO: 9), and the human CD30L gene was recovered from MT2 cells by PCR. The sequence was GenBank: BC111939.1 (SEQ ID NO: 10).

### [Experimental Example 2]

### (Confirmation of incorporation of mouse or human OX40L, 4-1BBL, GITRL, CD27L, and CD30L into BC-PIV)

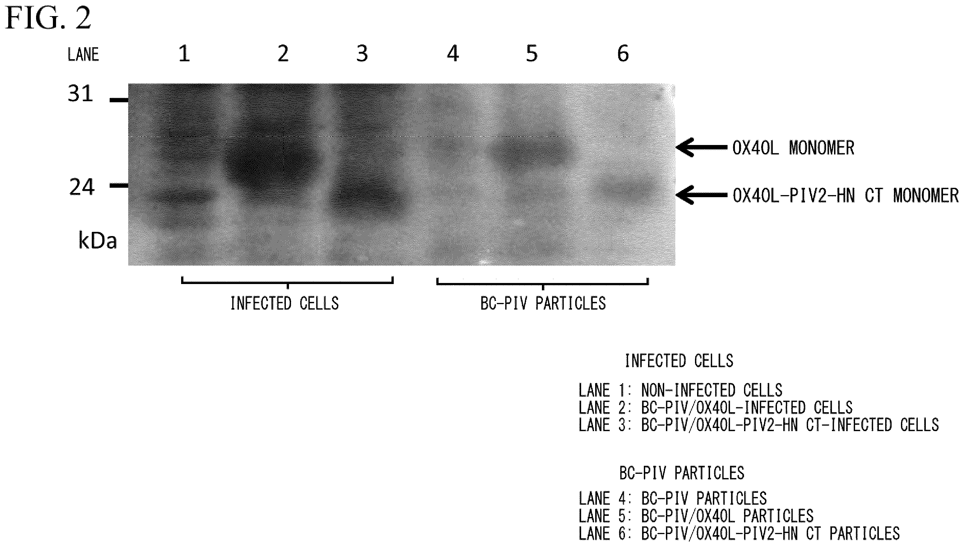

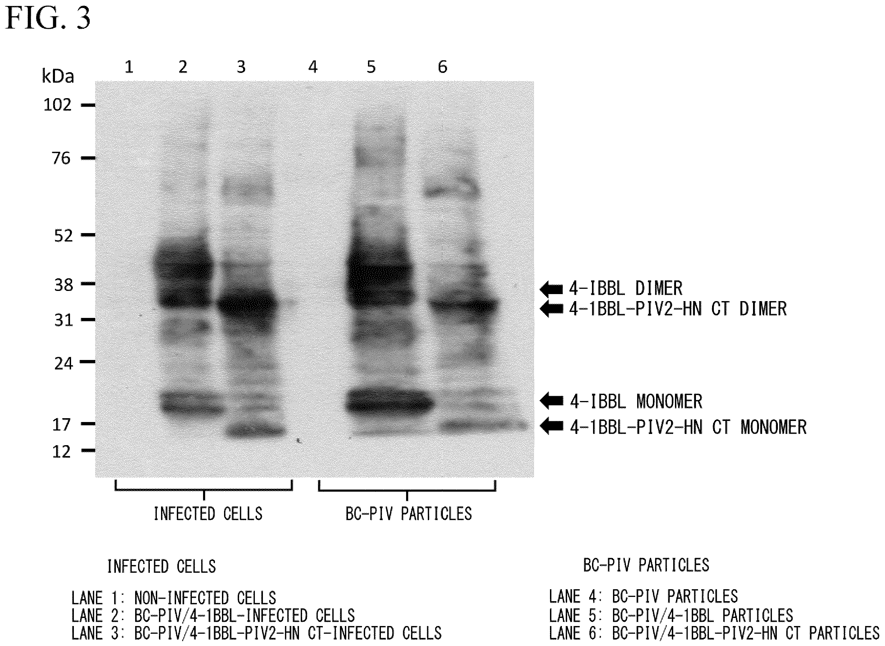

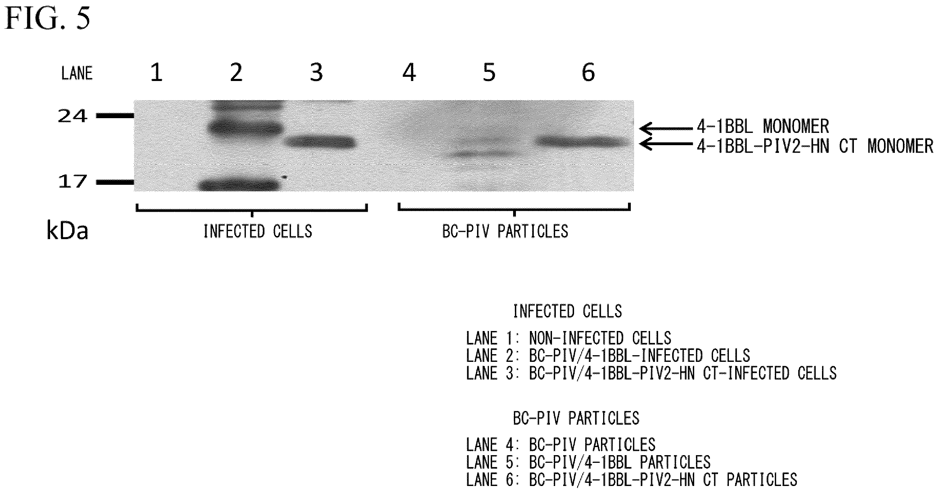

It was investigated whether the BC-PIV recovered in Experimental Example 1 expressed the target protein and whether the protein was incorporated into the BC-PIV. Since the BC-PIV carrying all the genes introduced into the NotI site and/or the Mlul site of the plasmid for virus preparation which was prepared in Experimental Example 1 was able to be recovered, the recovered BC-PIV was used to infect F membrane protein-expressing Vero cells of hPIV2 and cultured for 7 days, and recovered cells were subjected to Western blotting carried out using an anti-mouse OX40L antibody (R & D systems, cat #: BAF1236), an anti-mouse 4-1BBL antibody (R & D systems, R & D systems, cat #: AF1246), an anti-human OX40L antibody (ProSci Inc., cat #: 7243), an anti-human 4-1BBL antibody (Biorbyt Ltd, cat #: orb224047), an anti-mouse GITRL antibody (R & D systems, cat #: MAB21772), an anti-human GITRL antibody (R & D systems, cat #: AF694), an anti-mouse CD27L antibody (Sino Biological Inc, cat # 101956-T32), an anti-human CD27L antibody (Santa Cruz Biotechnology Inc, cat #: sc-365539), and an anti-human/mouse CD30L antibody (R & D systems, cat # MAB732) which are commercially available. The expression in the infected cells was described in Figs. 2, 3, and 5 based on the results of mouse OX40L and mouse and human 4-1BBL.

Next, the recovered BC-PIV was used to infect hPIV2 F membrane protein-expressing Vero cells, the cells were cultured for 7 days, and the supernatant was recovered in order to confirm whether OX40L, 4-1BBL, GITRL, CD27L, and CD30L were incorporated into the envelope of BC-PIV particles in which the desired gene was introduced into the NotI site of the plasmid for virus preparation.

The cell residues were removed from the supernatant by centrifugation at 3000 rpm (1700 xg), and impurities were further removed through a 0.8 µm filter. The obtained supernatant was ultracentrifuged (140000 xg, 40 minutes). After removal of the supernatant, the precipitate was suspended in PBS and subjected to Western blotting carried out using an antibody against the introduced protein as a BC-PIV virus fraction. The number of viruses used for Western blotting was set to 10⁶ particles.

In the subsequent Western blotting, in a case where the sample treatment was prepared using a sample buffer containing a reducing agent for typical Western blotting, antibody-positive signals were found in some cases not only in the monomer but also in the molecular weight considered as a multimeric structure because multimeric structures such as OX40L, 4-1BBL, GITRL, CD27L, and CD30L were strong.

### (Incorporation of mouse or human OX40L and 4-1BBL into viral vector particles)

Hereinafter, "incorporation of viral vector particles into the envelope" is described as "incorporation into viral vector particles" and "incorporation of BC-PIV particles into the envelope" is described as "incorporation into BC-PIV particles" unless otherwise specified.

Figs. 2 to 5 show the results of Western blotting for incorporation of mouse or human OX40L and 4-1BBL into viral vector particles.

Based on the results, mouse/human OX40L, mouse OX40L-PIV2-HN CT, mouse 4-1BBL, and mouse/human 4-1BBL-PIV2-HN CT were incorporated into BC-PIV particles. On the contrary, it was found that incorporation of human OX40L-PIV2-HN CT and human 4-1BBL into BC-PIV particles was difficult to be made.

### (Incorporation of mouse or human GITRL into viral vector particles)

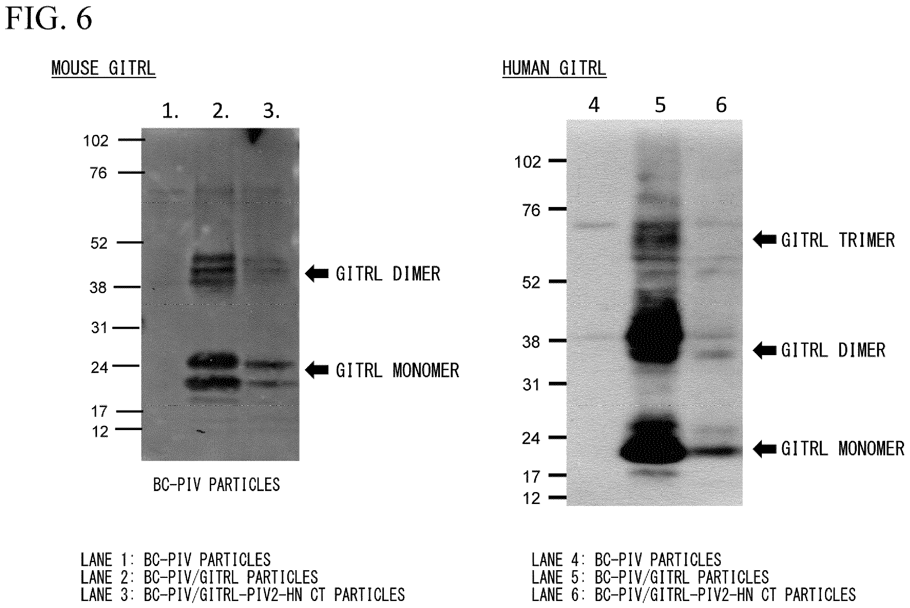

Fig. 6 shows the results of Western blotting for incorporation of mouse or human GITRL and GITRL-PIV2-HN CT into viral vector particles.

GITRL and GITRL-PIV2-HN CT were incorporated into BC-PIV particles in both mice and humans. However, the amount of human GITRL-PIV2-HN CT incorporated into the envelope membrane of BC-PIV particles was not so large. In the Western blotting, positive bands considered to be dimers in mice and dimers and trimers in humans were also confirmed in a case where the GITRL sample was treated with a sample buffer for western blotting, and the binding of multimers was shown to be strong.

### (Incorporation of mouse or human CD27L into viral vector particles)

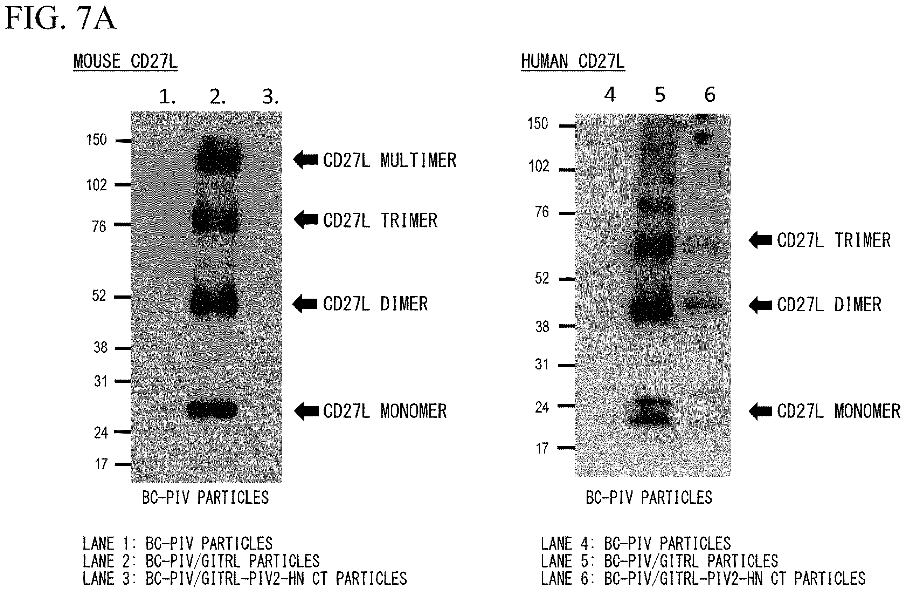

Fig. 7A shows the results of Western blotting of incorporation of mouse or human CD27L and CD27L-PIV2-HN CT into viral vector particles.

Both mouse CD27L and human CD27L were incorporated into BC-PIV particles. Meanwhile, only human CD27L-PIV2-HN CT was incorporated into the envelope membrane of BC-PIV particles. In both cases, positive bands were confirmed at the positions of the molecular weights considered to be multimers. In particular, almost no band was confirmed in the monomer in a case of human CD27L-PIV2-HN CT, and positive bands were recognized in the molecular weights considered to be dimers and trimers.

### (Incorporation of mouse or human CD30L into viral vector particles)

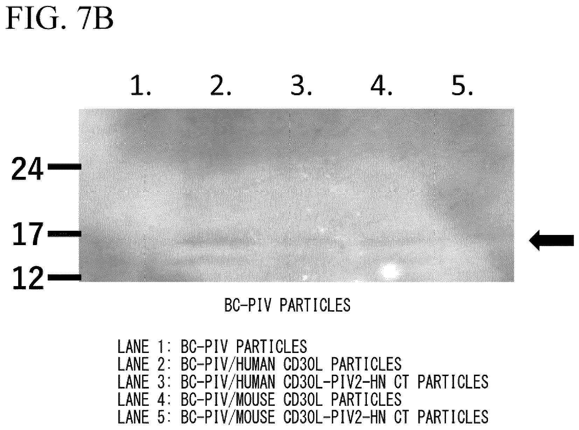

Further, even in a case of mouse CD30L and CD30L-PIV2-HN CT and human CD30L and CD30L-PIV2-HN CT, the band was not clear because the sensitivity of the antibody was not satisfactory, but a positive reaction was shown on BC-PIV vector particles, and incorporation into viral vector particles was recognized (see Fig. 7B).

As a result, OX40L, 4-1BBL, GITRL, CD27L, and CD30L or CT substitutes thereof were incorporated into the envelope membrane of BC-PIV particles, and OX40L, 4-1BBL, GITRL, CD27L, and CD30L that rapidly bind to the OX40 receptor, the 4-1BB receptor, the GITR receptor, the CD27 receptor, and the CD30 receptor that are inducibly expressed immediately after stimulation of T cells, NK cells, and the like can be provided by administration of the vector to solid tumors.

This can be considered as a property of an enveloped viral vector. That is, it can be said that the enveloped viral vector has an excellent characteristic that OX40L, 4-1BBL, GITRL, CD27L, or CD30L can be provided immediately after administration without undergoing the transcription and translation processes, which are different from an adenovirus vector that requires infection, transcription, and translation processes.

### [Experimental Example 3]

### (Construction of BC-PIV carrying two genes from OX40L, 4-1BBL, and GITRL genes)

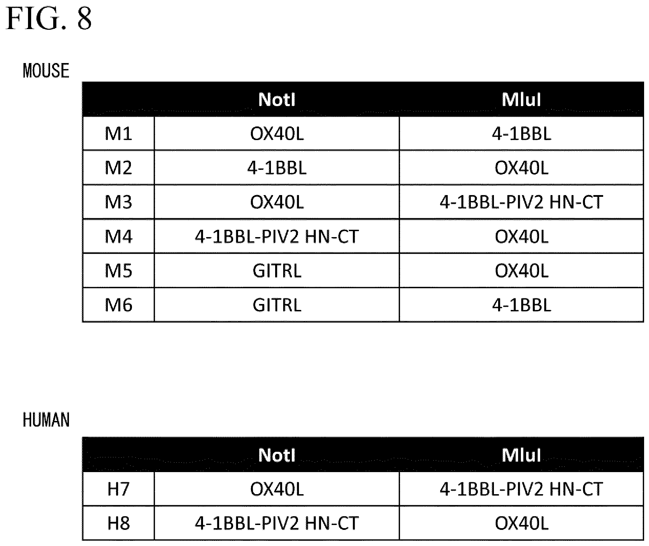

One TNF superfamily ligand gene has been introduced into BC-PIV so far. There are two sites where foreign genes can be introduced into the plasmid for BC-PIV virus preparation, which are the NotI and Mlul restriction enzyme sites. It is known that the expression level of hPIV2 is higher in the gene inserted into upstream NotI due to the expression polar effect. Therefore, the recovery of BC-PIV expressing the two genes was examined by introducing the OX40L gene, the 4-1BBL gene, and the GITRL gene into NotI and Mlul of the plasmid for virus preparation (Fig. 8).

As shown in Fig. 8, the combinations of the two genes and the insertion sites were set to be (1: M1) NotI: OX40L + MluI: 4-1BBL, (2: M2) NotI: 4-1BBL + MluI: OX40L, (3: M3) NotI: OX40L + MluI: 4-1BBL-PIV2-HN CT, (4: M4) NotI: 4-1BBL-PIV2-HN CT + MluI: OX40L, (5: M5) NotI: GITRL + MluI: OX40L, and (6: M6) NotI: GITRL + MluI: 4-1BBL in a case of mice.

In a case of humans, the combinations were set to be (7: H7) NotI: OX40L + MluI: 4-1BBL-PIV2-HN CT and (8: H8) NotI: 4-1BBL-PIV2-HN CT + MluI: OX40L.

The virus was recovered by the reverse genetics method described in Example 1. As a result, all BC-PIV viruses of (1) to (8) were able to be recovered.

### [Experimental Example 4]

### (Incorporation of two kinds of ligands into BC-PIV carrying two genes of OX40L and 4-1BBL genes)

The incorporation of the combination of M1-M4 shown in Fig. 8 ((1) NotI: OX40L + MluI: 4-1BBL, (2) NotI: 4-1BBL + MluI: OX40L, (3) NotI: OX40L + MluI: 4-1BBL-PIV2-HN CT, (4) NotI: 4-1BBL-PIV2-HN CT + MluI: OX40L) into BC-PIV was examined. 10⁶ particles recovered from the medium supernatant were subjected to Western blotting. The experiment was performed such that the used antibody and the dilution ratio were set to be the same as in the previous Western blotting test.

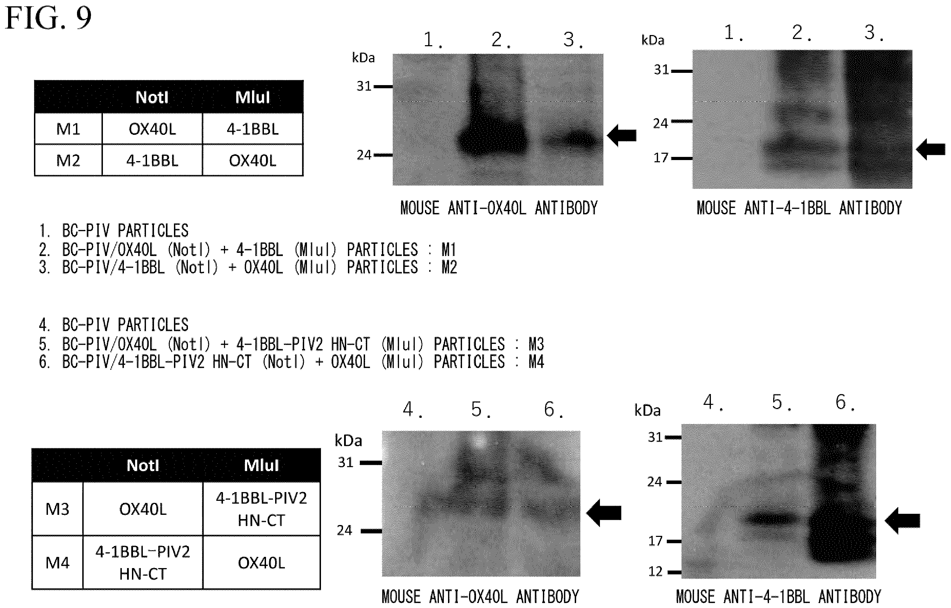

As can be seen in the results of Fig. 9, two proteins were incorporated into BC-PIV particles in the combination of M1-M4. Based on the incorporation amounts of proteins translated from the genes introduced into NotI and Mlul, the incorporation amount of the gene product introduced into NotI, which had a high expression level due to the polar effect, was higher.

### (Incorporation of ligand into BC-PIV carrying GITRL gene (NotI) and 4-1BBL gene or OX40L gene (Mlul))

The incorporation of the combination of M5-M6 shown in Fig. 8 ((5) NotI: GITRL + MluI: OX40L and (6) NotI: GITRL + MluI: 4-1BBL) into BC-PIV was examined.

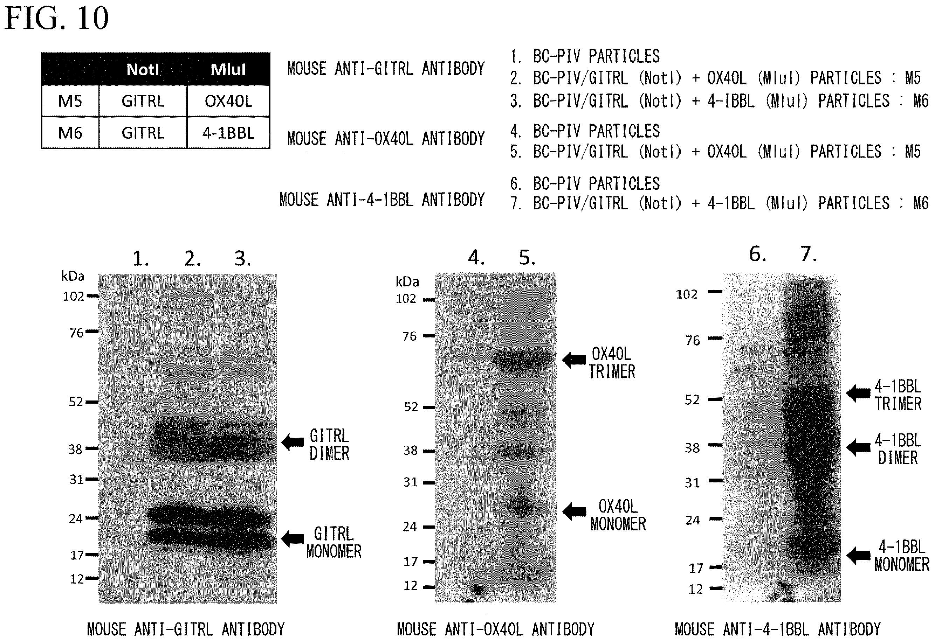

The test method was the same as described above. The results are shown in Fig. 10. As shown in the lanes 2 and 3 of Fig. 10, the incorporation of GITRL introduced into NotI in (5) and (6) into BC-PIV particles was confirmed. Further, the incorporation of OX40L (lane 5) introduced into Mlul in (5) and 4-1BBL introduced into Mlul in (6) into BC-PIV particles was also confirmed (lane 7).

Based on the results, GITRL, OX40L, and 4-1BBL were incorporated into BC-PIV particles as intended.

### (Incorporation of two kinds of ligands into BC-PIV carrying two genes of human OX40L gene and human 4-1BBL-PIV2-HN CT gene)

The incorporation of the combination of H7-H8 shown in Fig. 8 ((7) NotI: OX40L + MluI: 4-1BBL-PIV2-HN CT and (8) NotI: 4-1BBL-PIV2-HN CT + MluI: OX40L)) into BC-PIV was examined.

The test method was the same as described above.

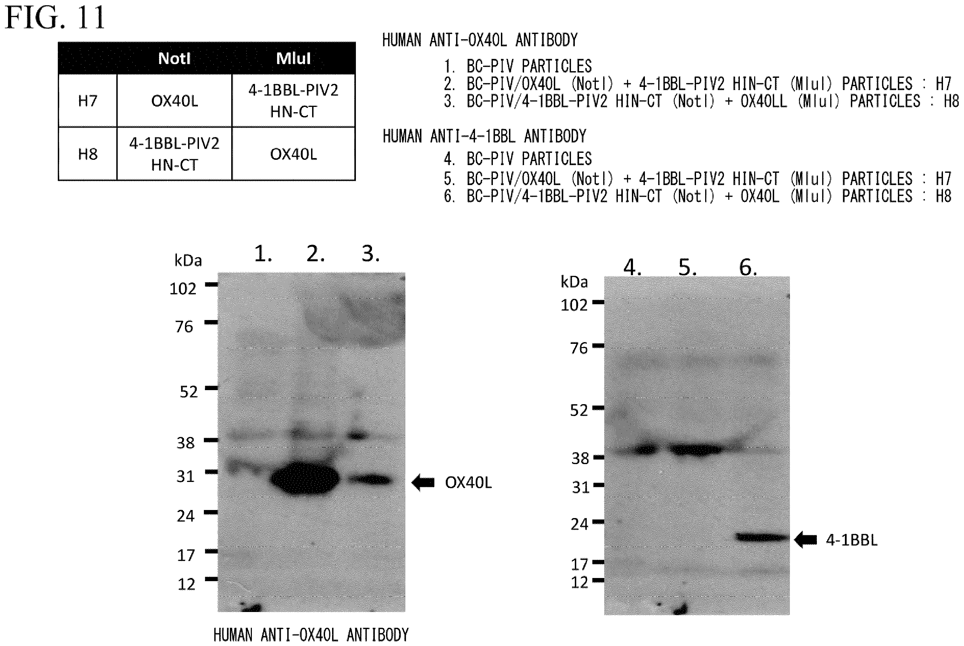

As shown in the lanes 2 and 3 of Fig. 11, human OX40L introduced into NotI and human OX40L introduced into Mlul in (7) were incorporated into BC-PIV particles. Quantitatively, the incorporation amount of human OX40L introduced into NotI was significantly higher. Meanwhile, as shown in the lanes 5 and 6 of Fig. 11, incorporation of human 4-1BBL-PIV2-HN CT was not able to be confirmed in a case of the sample introduced into Mlul of (7). Further, incorporation of the 4-1BBL-PIV2-HN CT sample introduced into NotI in (8) into BC-PIV particles was confirmed.

Based on the results, in mice, two kinds of proteins selected from the group consisting of OX40L, 4-1BBL, and GITRL were able to be incorporated into BC-PIV particles. In humans, since the incorporation was not made in some cases, each combination needs to be examined, and two TNFSF ligand proteins were able to be introduced into BC-PIV. That is, in vitro and in vivo, it is possible to obtain the effect of simultaneous activation of two types of TNFRSF signals in one cell.

### [Experimental Example 5]

### (Confirmation of binding of mouse and human OX40/4-1BB receptors and OX40L/4-1BBL on BC-PIV)

The confirmation of binding is important because binding of the OX40 receptor and the ligand or the 4-1BB receptor and the ligand triggers the activation of the TNFRSF signal.

1×10⁶ particles of BC-PIV considered to carry OX40L (mouse or human), OX40L-PIV2-HN CT (mouse or human), 4-1BBL (mouse only) or 4-1BBL-PIV2-HN CT (mouse only) was attached to 1 well of a 96-well ELISA plate overnight, a trimeric mouse OX40 receptor (C-terminal human IgG Fc Tag was added (BioLegend Inc, Cat # 78904), a human OX40 receptor (BioLegend Inc, Cat # 780804), or a mouse 4-1BB receptor (C-terminal human IgG Fc Tag was added (BioLegend Inc, Cat # 756704)) was added in an amount of 1, 10, 50, and 100 ng after treatment with Block Ace (Megmilk Snow Brand Co., Ltd.), Protein A (BioLegend Inc, Cat # 689202) conjugated with HRP adsorbed on the human Fc region was added, and the absorbance at 450 nm was measured using a POD substrate TMB kit (Nakaraitesk). The test was performed four times consecutively for each case.

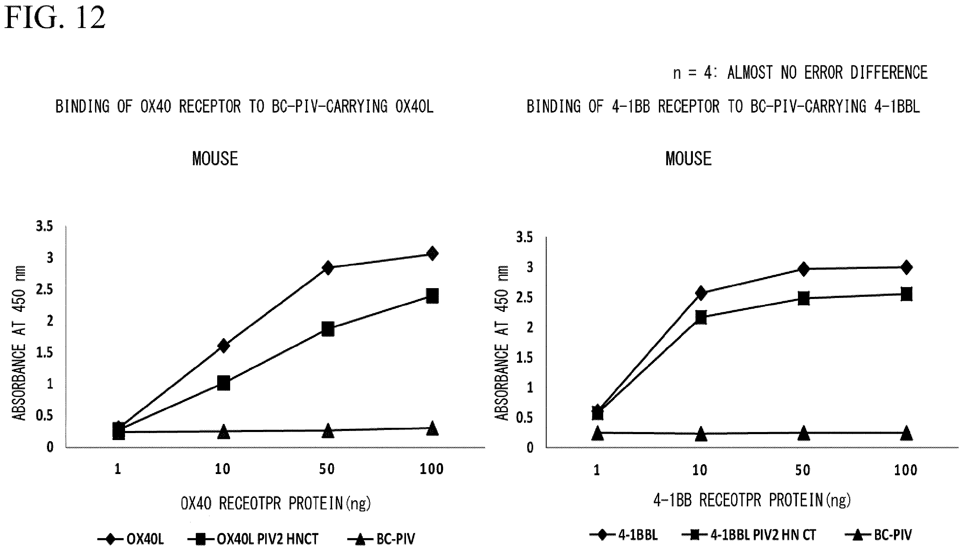

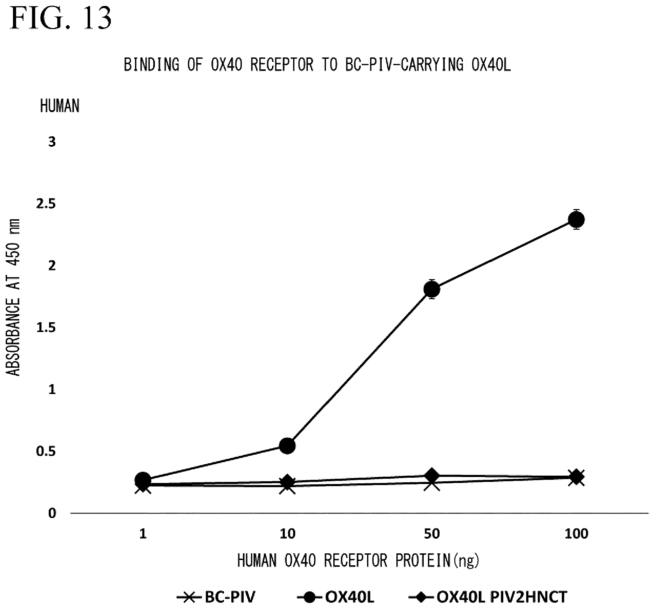

Fig. 12 shows the results of the mice, and Fig. 13 shows the results of the humans.

First, the results of mice are shown. The absorbance of OX40L and OX40L-PIV2-HN CT incorporated into BC-PIV increased depending on the addition concentration of the OX40 receptor, and adsorption on OX40 was found. The binding ability of OX40L was higher than that of OX40L-PIV2-HN CT (Fig. 12, left graph).

The absorbance of 4-1BBL and 4-1BBL-PIV2-HN CT also increased depending on the addition concentration of the 4-1BB receptor, but equilibrium was shown in a case where the addition amount was greater than 10 ng. The binding of 4-1BBL was higher than that of 4-1BBL-PIV2-HN CT (Fig. 12, right graph).

It is assumed that the numbers of OX40L and 4-1BBL proteins incorporated into 1×10⁶ particles of BC-PIV are similar, the binding ability of the 4-1BBL protein incorporated into BC-PIV is assumed to be higher than that of OX40L.

Based on the results, it was suggested that the TNFSF ligand incorporated into BC-PIV is likely to bind to the TNFSF receptor and activate the signal.

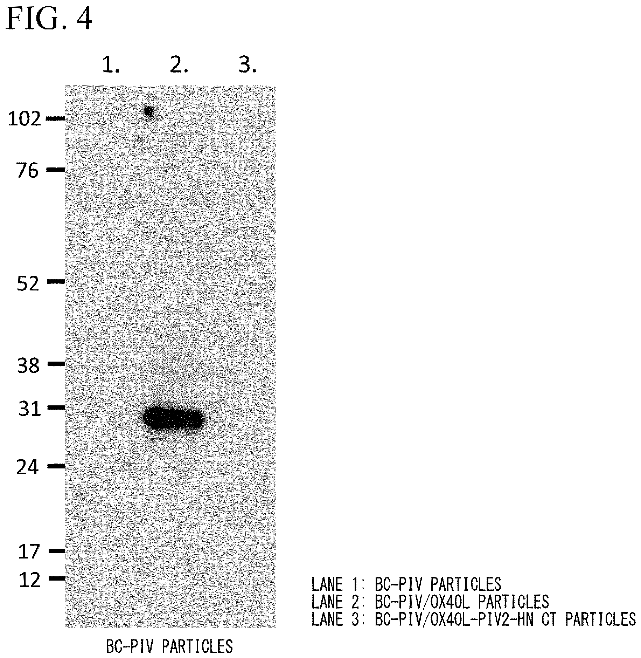

Next, the results of humans are shown in Fig. 13. The absorbance of OX40L incorporated into BC-PIV was increased depending on the addition concentration of the OX40 receptor, and adsorption on OX40 was found. However, OX40L-PIV2-HN CT did not almost react. This is consistent with the result that OX40L-PIV2-HN CT is not incorporated into the virus particles, as shown in Fig. 4.

Based on the results, it was confirmed that the human TNFSF ligand incorporated into BC-PIV particles also binds to the TNFSF receptor, which indicates the possibility of extrapolation of mouse results to humans.

### (Confirmation that protein incorporated into BC-PIV is multimer)

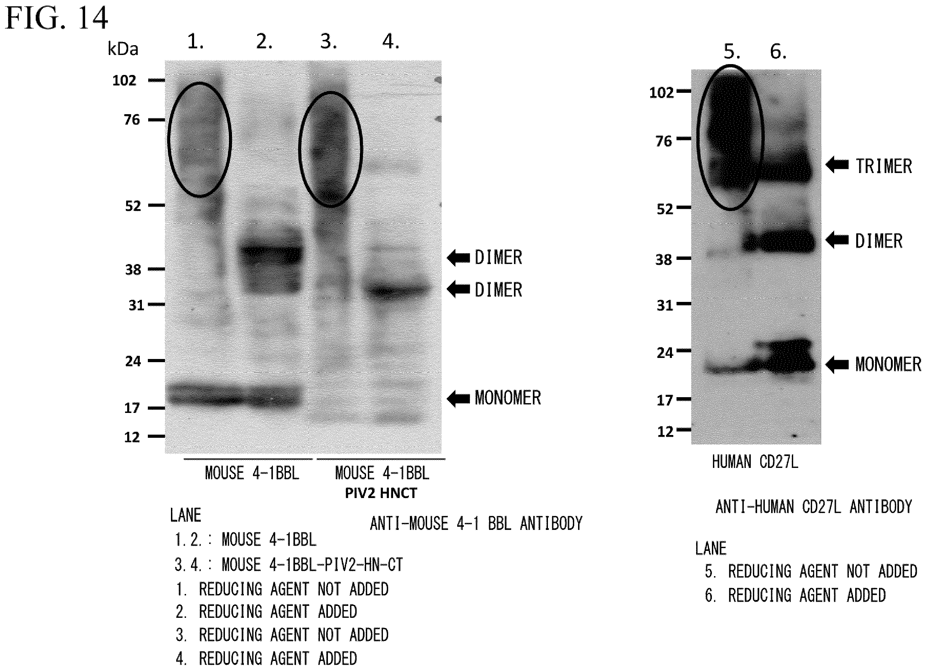

Mouse 4-1BBL, 4-1BBL-PIV2-HN CT, and human CD27L incorporated into BC-PIV were treated with a sample buffer in the presence or absence of a reducing agent (DTT), and the state of 4-1BBL, 4-1BBL-PIV2-HN CT, and CD27L was investigated (Fig. 14). 4-1BBL, 4-1BBL-PIV2-HN CT, and CD27L in a state where no reducing agent was added during the sample recovery were shifted from a state of a high-molecular weight (even lane O) with associated multimeric structures to a state of a low-molecular weight (odd lane) with broken multimeric structures, by addition of DTT. Based on the results, mouse 4-1BBL, 4-1BBL-PIV2-HN CT, and human CD27L were expected to form high-molecular-weight multimers on BC-PIV particles (basically, trimers are formed, and the trimers are further associated).

### [Experimental Example 6]

### (Purification of Pantoea agglomerans LPS isolated from Zizania latifolia (extraction using heat (121°C for 20 minutes) and cold EtOH))

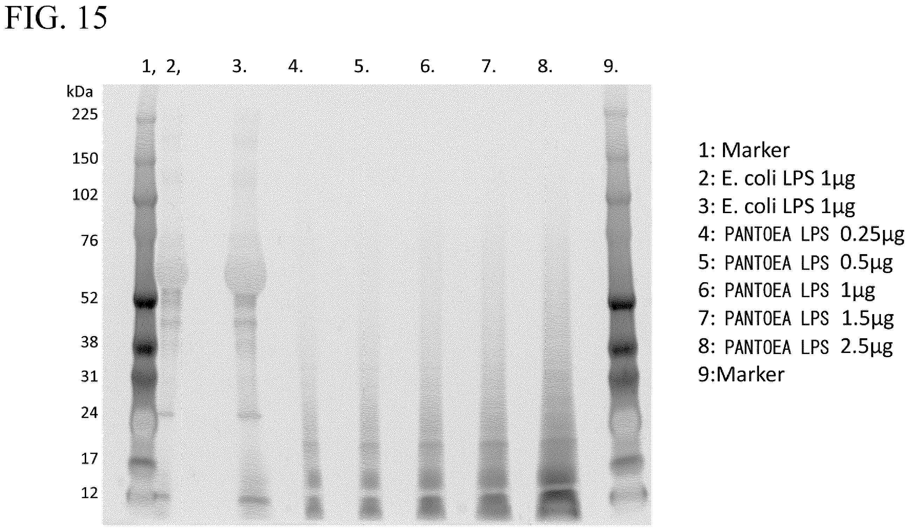

3.5 g of bacterial cells obtained by culturing Pantoea agglomerans isolated from Zizania latifolia was suspended in 50 mL of PBS, autoclaved (121°C for 20 minutes), and cooled to room temperature. A centrifugal treatment (10000 rpm (9100 xg) for 30 minutes) was performed, and the supernatant was recovered. 3 mL of 5M sodium chloride and 27 mL of 100% ethanol were added to 20 mL of the supernatant, and the mixture was allowed to stand overnight at -80°C. The mixture was thawed at room temperature, centrifuged at 4°C (9100 xg) for 30 minutes to remove the supernatant, suspended in 5 mL of 70% ethanol, centrifuged at 10000 rpm (9100 xg) at 4°C for 20 minutes to remove the supernatant, and sufficiently dried. The resultant was dissolved in 10 mL of distilled water and used in the following test. 0.5 µL, 1 µL, 2 µL, 3 µL, and 5 µL of the sample solutions were electrophoresed with 1 µg and 3 µg of LPS of Escherichia coli, and silver staining was performed (see Fig. 15). The main characteristic of LPS extracted by heat (121°C for 20 minutes) and cold EtOH was the lower molecular weight as compared with LPS of Escherichia coli. The molecular weight was different from the molecular weight of LPS in Escherichia coli. With reference to LPS of Escherichia coli, the concentration was estimated to be approximately 0.5 µg/µL.

### (Characteristics of LPS)

Most of the samples recovered this time are products considered to be low-molecular-weight LPS (see Fig. 15). It is considered that high-molecular-weight LPS is more toxic.

TNFα produced by macrophages activated by LPS activates neutrophils, and esterase damages vascular endothelial cells. Further, hemorrhagic necrosis is induced with respect to cancer. In addition, LPS promotes blood coagulation and causes microcirculatory disorders, which causes DIC (disseminated intravascular coagulation). However, a low dose of TNFα is considered to be useful cytokines for antitumor immunity.

While LPS induces cytokines such as TNFα, LPS has the ability to activate dendritic cells and NK cells. Innate immunity of NK cells and the like is considered to be important for the antitumor immunity in addition to acquired immunity of CTL and the like specific to cancer cells, and NK cell activation by low-molecular-weight LPS was examined in order to examine the synergistic effect of NK cell activation by the LPS in addition to acquired immunity of CTL and the like by TNFRSF signals.

### (Activation of NK cells due to LPS extracted using heat (121°C for 20 minutes) and cold EtOH method)

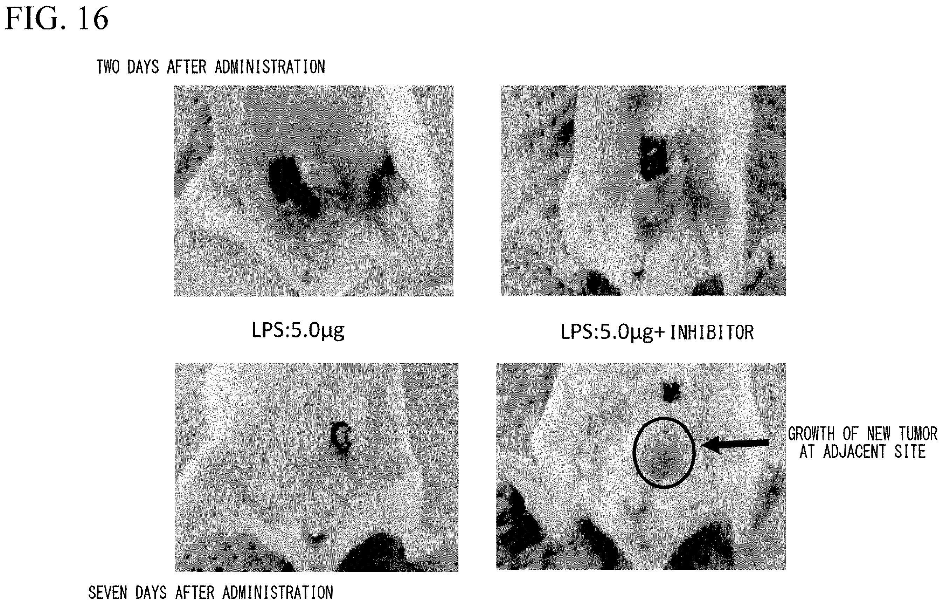

The antitumor effects obtained by administering an extract of LPS extracted using heat (121°C for 20 minutes) acquired from Pantoea agglomerans and the cold EtOH method to CT26 mouse colorectal cancer tumors transplanted into a mouse, alone or together with an anti-asialo GM1 antibody which is an inhibitor of NK cells were investigated. After depilation of the ventral side of the mouse, 5×10⁵ CT26 mouse colorectal cancer cells were inoculated into the ventral subcutaneous (SC) site under anesthesia. 100 µL (Wako Pure Chemical Industries, Ltd.; code # 014-09801) of the anti-asialo GM1 antibody was intraperitoneally administered 3 days before the administration of LPS. In a case where the major axis of the tumor reached 5 to 7 mm, 5 µg (10 µL) was inoculated into the tumor. The state of the tumor after 2 and 7 days from the administration is shown in Fig. 16. After administration, the mice exhibited symptoms such as nap. Two days after administration, the tumor region turned black. Seven days after the administration, the black region of the tumor site shrank, but in the LPS + anti-asialo GM1 antibody-administered mice, new tumor growth was observed in the vicinity of the necrotic tumor (see the O part on the lower right side of Fig. 16). Meanwhile, growth of new tumor was not recognized in the mice to which only LPS had been administered. It was shown that the LPS has the potential to induce NK cells.

### [Experimental Example 7]

(BC-PIV/OX40L in which OX40L gene is introduced into Mlul site of plasmid for BC-PIV virus preparation, antitumor effects on CT mouse 26 colorectal cancer by administration of anti-mouse OX40 agonist antibody)

The point of the present experimental example is to investigate the tumor regression effect at the administration site and the distant site by inoculating CT26 cells into two sites and administering BC-PIV, BC-PIV/OX40L, and BC-PIV + anti-mouse OX40 agonist antibodies to solid cancer at one site between the two sites.

It was reported that the anti-mouse OX40 agonist antibody has no effect in a case of being used alone (see Non Patent Document 9), and thus the anti-mouse OX40 agonist antibody was not administered alone here.

After depilation of the ventral side of the mouse, 1×10⁶ CT26 mouse colorectal cancer cells were inoculated into two ventral subcutaneous (SC) sites under anesthesia. Six days later, animals whose major axis of tumor reached 5 to 7 mm were divided into the following groups (3 animals for each group), and the sample was administered to one of the two cancer sites. The administration was performed 3 times every other day.

Group 1: PBS administration group
Group 2: BC-PIV administration group
Group 3: BC-PIV/OX40L administration group
Group 4: BC-PIV + anti-mouse OX40 agonist antibody administration group Each sample was prepared in an amount of 60 µL and administered.

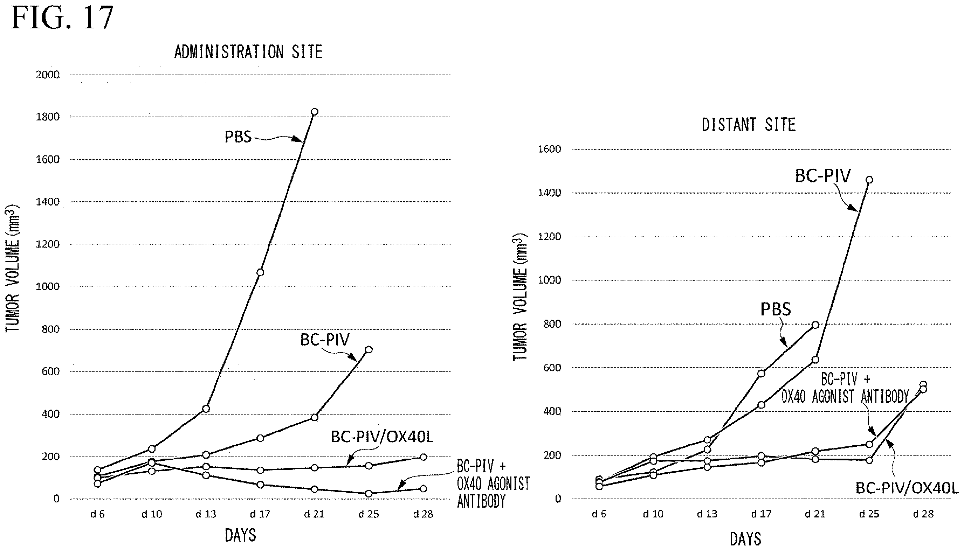

The results of changes in tumor volume with time are shown in Fig. 17. The average value of two animals and the average value of three animals were respectively used for PBS. After the third administration, the tumor diameter was measured every 3 days or 4 days, and the tumor volume was acquired by calculating "minor axis x minor axis x major axis x 0.5". Animals whose major axis of tumor reached approximately 15 mm were treated as ethically dead.

The administered viral load of BC-PIV was 5.3×10⁷ particles. A virus recovered by introducing the OX40L gene into the Mlul restriction enzyme site of a plasmid for BC-PIV preparation was used for BC-PIV/OX40L. The viral load of BC-PIV/OX40L was 6.5×10⁷ particles. 5 µg of the anti-mouse OX40 agonist antibody (BioLegend Inc., cat #: 119408) was used.

The results are shown in Fig. 17. In two of the three animals in the BC-PIV + anti-mouse OX40 agonist antibody administration group, the tumor volume at the administration site reached 0 and disappeared. Tumors almost regressed in two of three animals in the BC-PIV/OX40L administration group. At the site of direct administration to the tumor, a large difference in tumor volume between the PBS administration group and the BC-PIV administration group was recognized, and the antitumor activity even only in the BC-PIV administration group was recognized.

In addition, there was a clear difference in tumor volume between the BC-PIV administration group and the BC-PIV/OX40L administration group, and the effect of OX40L incorporated onto BC-PIV was considered to be large. There was not much difference in tumor volume between the BC-PIV/OX40L administration group and the BC-PIV + anti-mouse OX40 agonist antibody administration group, and tumors of two animals in the BC-PIV + anti-mouse OX40 agonist antibody administration group disappeared and tumors of two animals in the BC-PIV/OX40L administration group also significantly regressed.

As for the distant tumors, the proliferation inhibitory effect was clearly recognized in the BC-PIV/OX40L and BC-PIV + anti-mouse OX40 agonist antibody administration groups as compared with other groups (see Fig. 17).

In Sci. Transl. Med. (See Non Patent Document 9), Idit Sagiv-Barfi et al. indicate that almost no antitumor inhibitory effect was recognized at the administration site and the distant site in a case where only the anti-mouse OX40 agonist antibody was administered, OX40 is induced in CD4-positive T cells by the combined administration with CpGDNA, and a high antitumor effect can be obtained at the administration site and the distant site by combining OX40 and CpGDNA. It was considered that since the tumor inhibitory effect was recognized in a case of using the combination of BC-PIV and the anti-mouse OX40 agonist antibody, only BC-PIV is effective in inducing the expression of OX40 in CD4-positive T cells.

### [Experimental Example 8]

### (Antitumor effect of BC-PIV, anti-mouse OX40 agonist antibody, and LPS on CT26 colorectal cancer)

Further, in Sci. Transl. Med. (See Non Patent Document 9), Idit Sagiv-Barfi et al. indicate that a rapid and significant increase in number of NK cells in the tumor (cancer) tissues is recognized after administration of the CpG DNA + anti-mouse OX40 agonist antibody to solid cancer, and thus a significant increase in the number of NK cells leads to suppression of proliferation of cancer cells.

Further, activation of dendritic cells that present a tumor cell-specific CTL epitope is also important for CTL activation. The inventors previously reported that LPS derived from plant Zizania latifolia symbiotic Pantoea agglomerans strongly matures dendritic cells (see Patent Document 3). Further, Fig. 16 shows that LPS alone can activate NK cells.

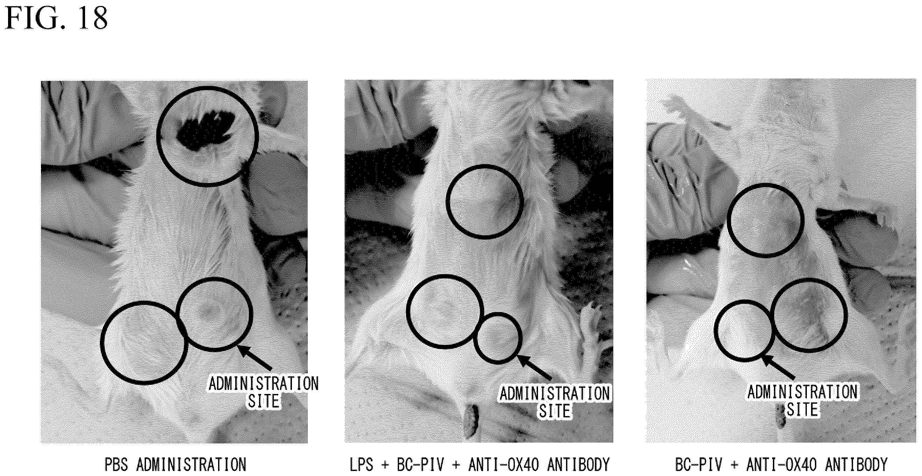

Therefore, the combination of LPS, BC-PIV, and the anti-mouse OX40 agonist antibody and the antitumor effects were investigated. After depilation of the ventral side of the mouse, CT26 mouse colorectal cancer cells were inoculated into three ventral (5×10⁵ cells (one site), 3×10⁵ cells (two sites)) subcutaneous (SC) sites under anesthesia. Five days later, the sample was administered to one of the three cancers where the major axis of the tumor reached 5 to 7 mm. The administration was performed 3 times every other day. A combination of LPS, BC-PIV, and the anti-mouse OX40 agonist antibody extracted from Pantoea agglomerans using heat (121°C for 20 minutes) and the cold EtOH method was administered. Fig. 18 shows the results of combined administration of LPS (2.5 µg), BC-PIV (5×10⁷ particles), and the anti-mouse OX40 agonist antibody (5 µg), after 30 days from the administration which is the most effective timing.

Compared to the PBS administration, the BC-PIV + anti-mouse OX40 agonist antibody combination administration and the LPS + BC-PIV + anti-mouse OX40 agonist antibody combination administration (one of three mice) (see the mouse in the middle of Fig. 18) showed higher tumor inhibitory effects. In particular, in the latter combination, large tumors were selected and administered at the time of administration.

### (Effect of LPS)

Since the effect of LPS was suggested in Fig. 16, the effect of LPS was investigated with a combination of LPS and BC-PIV/OX40L in which the OX40L gene was introduced into the Mlul site of the plasmid for virus preparation. The number of administered particles of BC-PIV/OX40L was set to 1.6×10⁷, and the number of administrations was set to 2 times, which is one less than the typical administration. Administration was performed by dividing animals into a PBS administration group (control group), a BC-PIV/OX40L + LPS (0 µg) administration group, a BC-PIV/OX40L + LPS (2.5 µg) administration group, a BC-PIV/OX40L + LPS (5 µg) administration group, a BC-PIV/OX40L + LPS (12.5 µg) administration group, and an LPS (12.5 µg) administration group. The number of animals was all set to "n = 3", and the tumor volume was shown based on the average value thereof. In a case where two of the three mice were considered dead, the group was excluded.

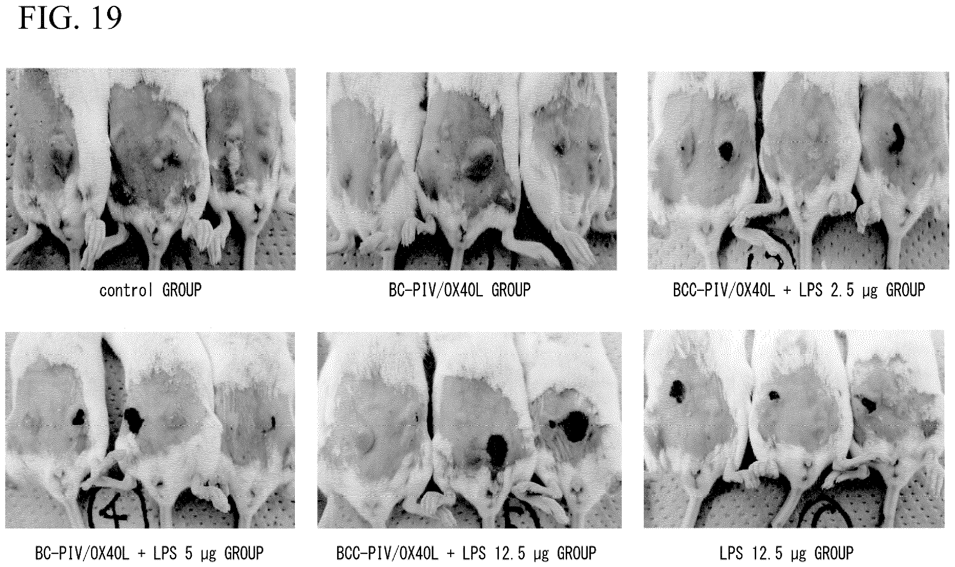

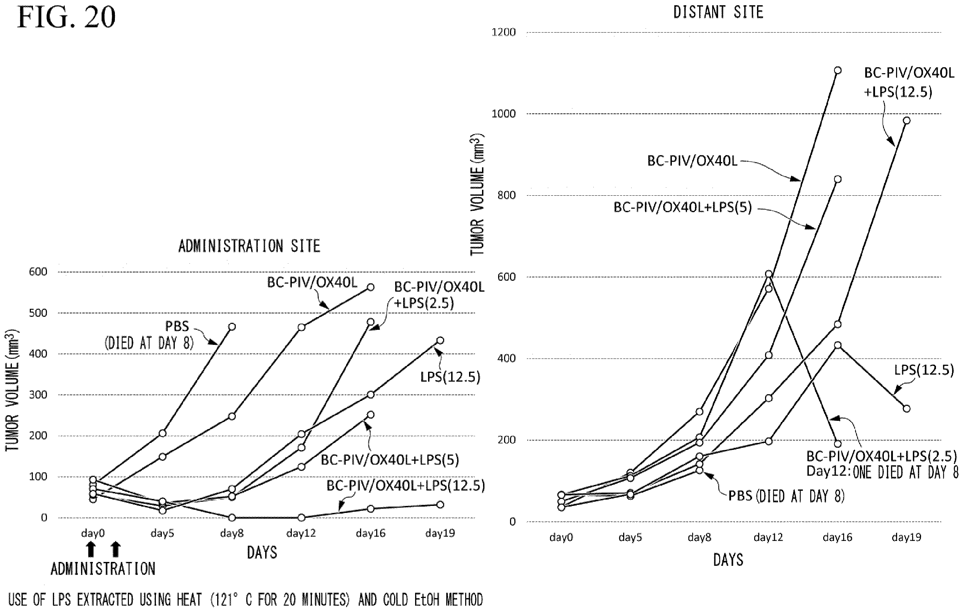

Fig. 19 shows the state of the tumors at the time of the second administration, and Fig. 20 shows the results of the tumor volumes. It was confirmed that the tumor site to which LPS had been administered turned black, and in a case where a combination of BC-PIV/OX40L and LPS was administered, the proliferation of tumors at the administration site was suppressed depending on the amount of LPS (see the left graph in Fig. 20). Tumors of two mice in the BC-PIV/OX40L + LPS (10 µg) group, two mice in the BC-PIV/OX40L + LPS (12.5 µg) group, and one mouse in the LPS (12.5 µg) group on the administration side disappeared. Only in the BC-PIV/OX40L + LPS (12.5 µg) group, mice did not die until the day 19. The distant cancer of one mouse of the BC-PIV/OX40L + LPS (10 µg) group and one mouse of the LPS (12.5 µg) group, with a relatively small tumor diameter (3.2×5.2 mm and 3.2×3.5 respectively) at the time of administration, regressed at both the administration site and the distant tumor. The control group died prematurely due to a large increase in tumor volume at the administration site. The proliferation of tumors in other groups was not significant as compared to that of the control group.

In addition, there was a slight difference in volume of distant tumors due to the exclusion of death, but the influence on the shrinkage of tumor at a distant site depending on the LPS concentration was considered to be small (see Fig. 20). The PBS administration group was excluded early due to a rapid increase in tumor volume.

In the present test, the number of times of administration of BC-PIV/OX40L was decreased, and the details of the dosage volume of LPS were investigated. In the LPS administration, as shown in the results on the third day from the first administration in Fig. 19, the administered tumor site turned black from the day after the first administration, and the tumor turned extremely small two days after the administration. In a case where the dose of LPS was large, the tumor shrank in a case of the second administration, and most of the sample was not able to be effectively administered into the tumor in some cases.

In the HE-stained image of the tumor tissues 1 day after the administration of BC-PIV/OX40L + LPS (5 µg), it was observed that a large number of mononuclear round cells whose morphology was completely different from that of the bleeding marks and the tumor cells were infiltrated and proliferated. Necrobiosis lesions with scattered microhemorrhage were recognized in the central portion of the tumor, and many macrophages phagocytosing hemosiderin were also observed, and thus it was expected that rapid cancer cell hemorrhagic necrosis was induced by a large amount of TNFα induced by LPS. However, it was found that since there was a difference in proliferation of tumor at the administration site between the LPS (12.5 µg) administration group and the BC-PIV/OX40L + LPS (12.5 µg) administration group, the effect of BC-PIV/OX40L was also exhibited.

Meanwhile, in cancers at distant sites, since the number of times of administration of BC-PIV/OX40L was only twice, which was small, the effect was not as high as the typical three administration, and the impact of the volume of LPS on the antitumor effect at distant sites was not almost recognized. Based on the test results, since hemorrhagic necrosis of the tumor was recognized in the administration of the dose of LPS used here, it was considered necessary to reduce the dose of LPS in a case where LPS was added.

### [Experimental Example 9]

### (Influence of OX40L on NK cells and CD4-positive cells and CD8-positive T cells and effects of 4-1BBL)

Idit Sagiv-Barfi et al. indicate a rapid increase of 4-1BB (CD137) in NK cells accumulated in the tumor, in the report of the antitumor effects of CpG DNA and the anti-mouse OX40 agonist antibody, at the administration site and the distant site, which were administered simultaneously to solid tumors. Therefore, in the present test, the effect of BC-PIV into which OX40L and 4-1BBL-PIV2-HN CT were introduced was confirmed, and the influence of BC-PIV on the proliferation of tumors was investigated using an inhibitor (anti-asialo GM1 antibody for NK cells and anti-mouse CD4/8 antibody for CD4/8-positive T cells) in order to investigate the involvement of NK cells and CD4/8-positive T cells in antitumor immunity.

BC-PIV/OX40L or BC-PIV/4-1BBL-PIV2-HN CT was prepared by introducing a predetermined gene into the Mlu site (see Fig. 1) of the plasmid for BC-PIV preparation. The total number of BC-PIV particles used for administration was set to 8.1×10⁶ particles. The administration was performed 3 times every other day. The dose was fixed at 7.5 µg using LPS extracted from Pantoea agglomerans using heat (121°C for 20 minutes) and cold EtOH. The dosage volume of the sample was set to 60 µL.

After depilation of the ventral side of the mouse, 5×10⁵ CT26 mouse colorectal cancer cells were inoculated into two ventral subcutaneous (SC) sites under anesthesia. One week later, animals whose major axis of tumor reached 5 to 7 mm were divided into the following groups (3 animals for each group), and the sample was administered to one of the two cancer sites.

The administration was performed by dividing animals into the following 12 groups. The tumor diameter was measured with the day of the first administration set as the day 0. For NK cell suppression, an anti-asialo GM1 antibody (Wako Pure Chemical industries Ltd., 100 µL/animal) was administered intraperitoneally 3 days before the sample administration, and an anti-CD4/8 antibody (anti-CD4 antibody: Tonbo biosciences, Cat #: 70-0041, 100 µg/animal, anti-CD8 antibody: Bio X cell, Cat #: BP0061, 100 µg/animal) was intraperitoneally administered the day before and the day after the first sample administration.

Group 1: PBS administration group
Group 2: LPS (7.5 µg) administration group
Group 3: BC-PIV/OX40L (half dose) + BC-PIV/4-1BBL (half dose) + LPS (7.5 µg) administration group
Group 4: OX40 agonist antibody + LPS (7.5 µg) administration group
Group 5: BC-PIV/OX40L administration group
Group 6: BC-PIV/4-1BBL-PIV2-HN CT administration group
Group 7: BC-PIV/OX40L + LPS (7.5 µg) administration group
Group 8: BC-PIV/4-1BBL-PIV2-HN CT + LPS (7.5 µg) administration group
Group 9: BC-PIV/OX40L + LPS (7.5 µg) + anti-NK antibody administration group
Group 10: BC-PIV/4-1BBL-PIV2-HN CT + LPS (7.5 µg) + anti-NK antibody administration group
Group 11: BC-PIV/OX40L + LPS (7.5 µg) + anti-CD4/8 antibody administration group
Group 12: BC-PIV/4-1BBL-PIV2-HN CT + LPS (7.5 µg) + anti-CD4/8 antibody administration group

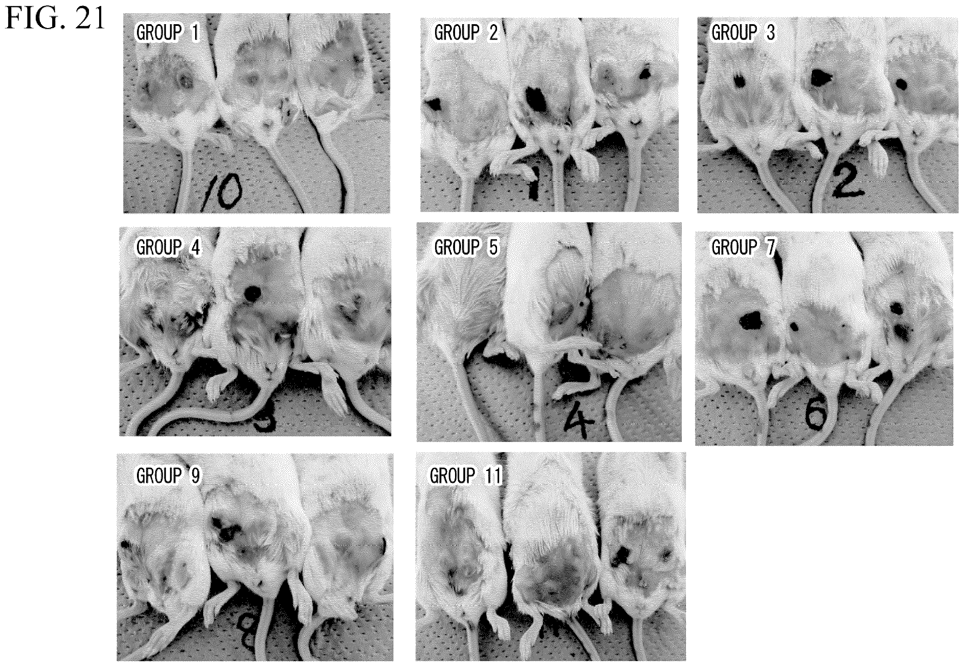

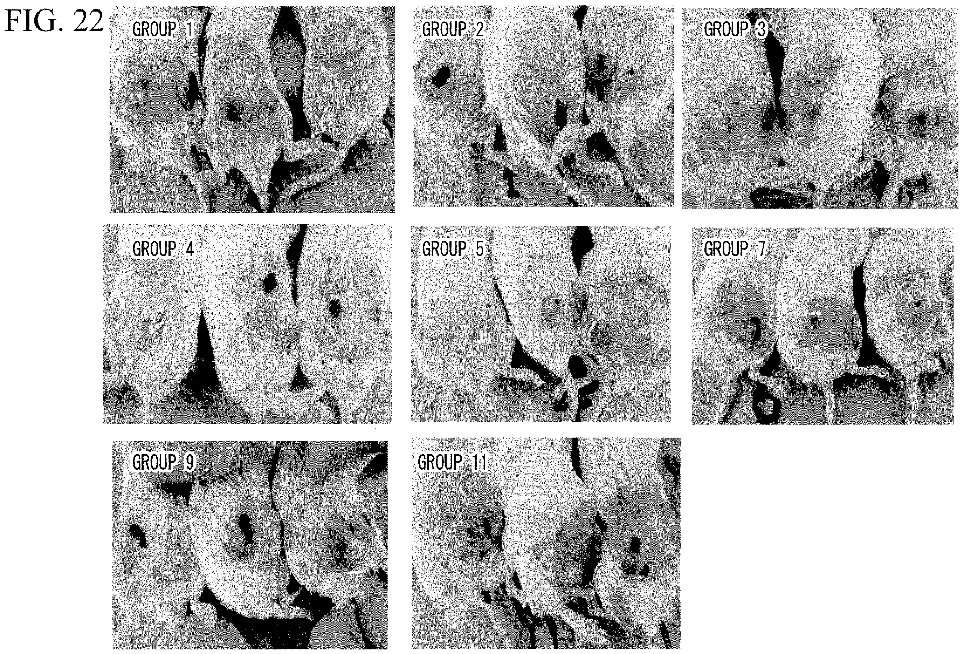

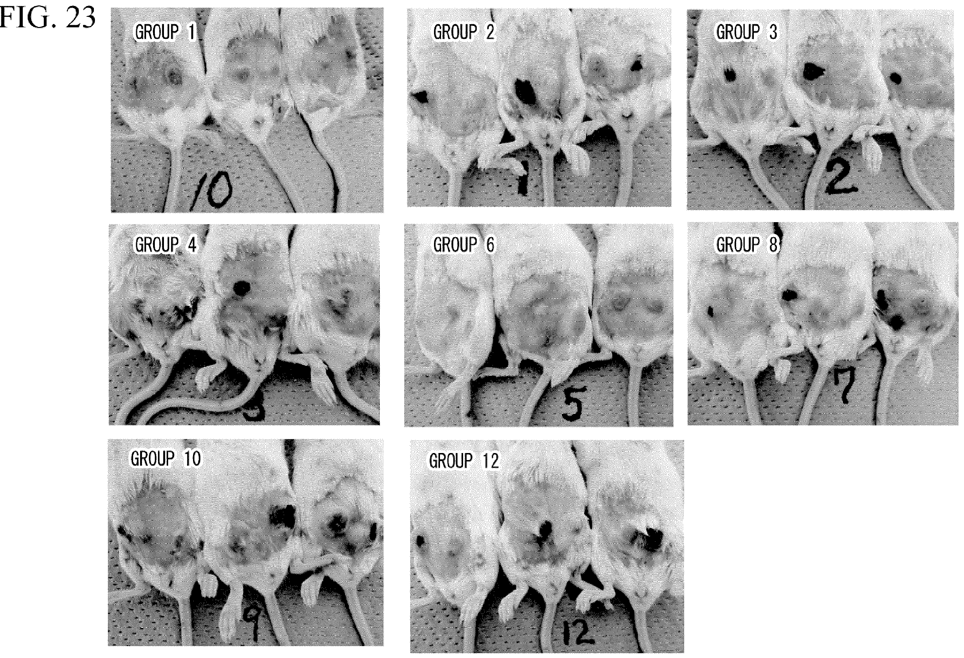

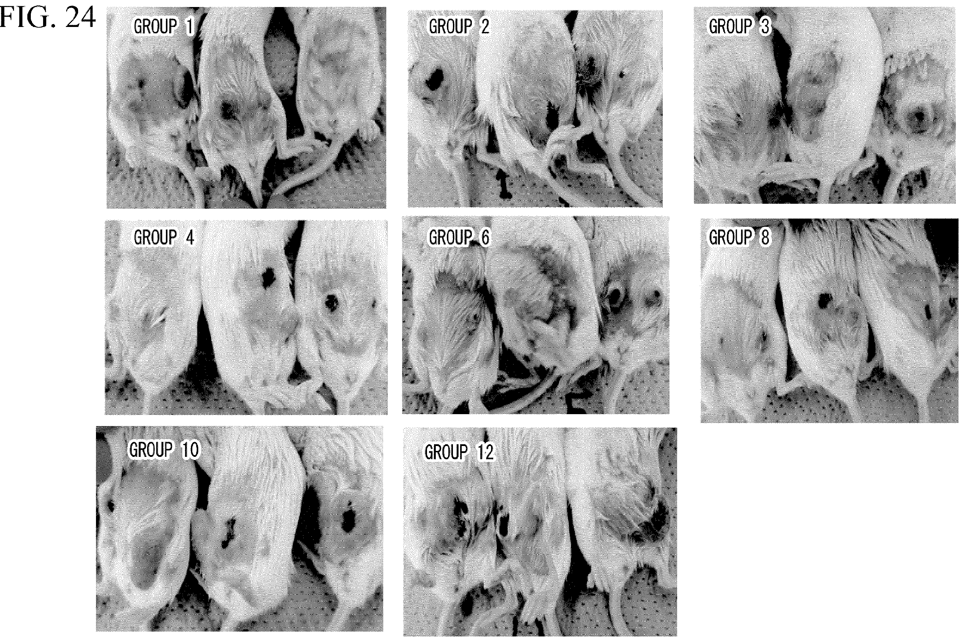

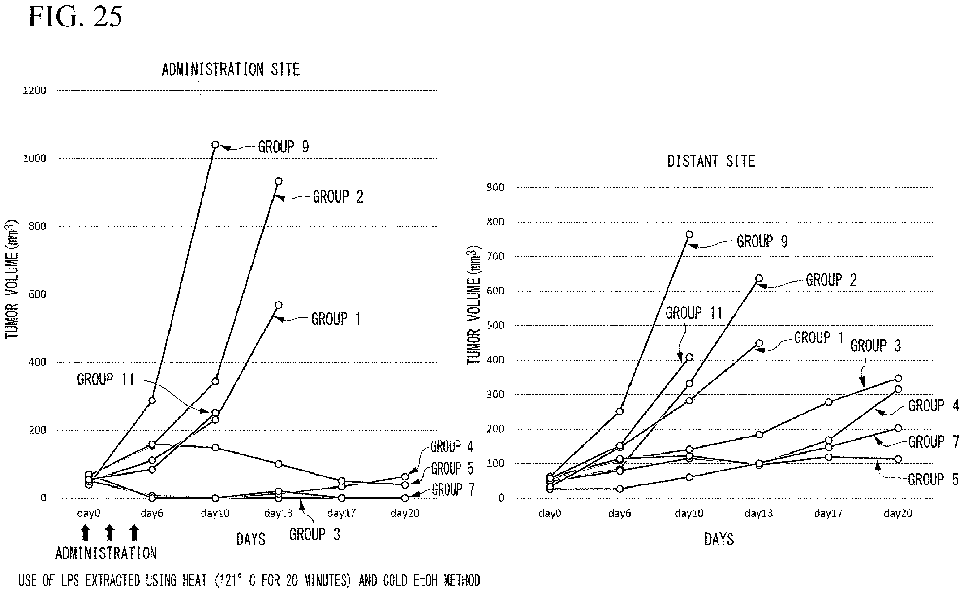

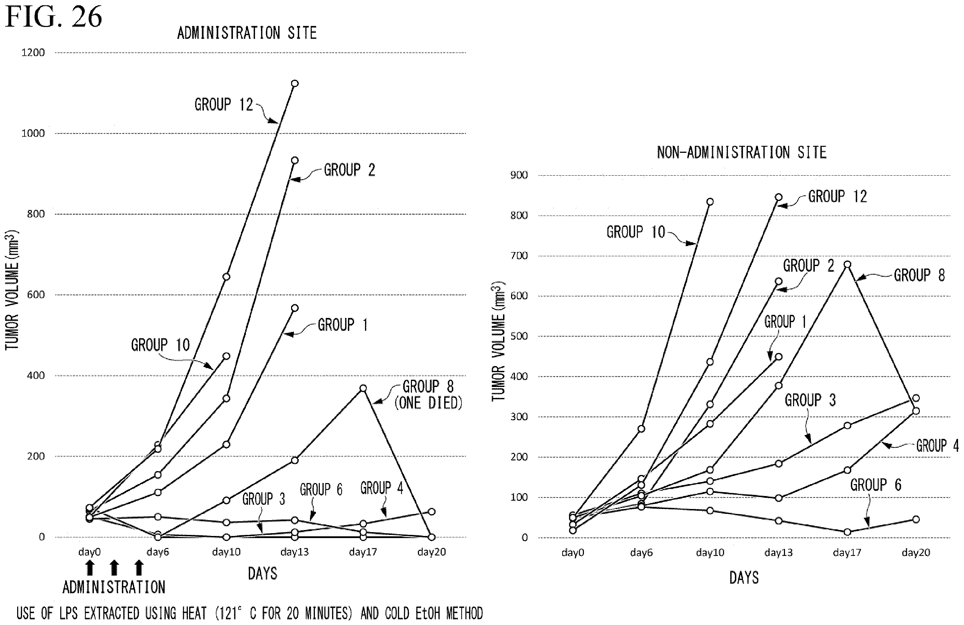

As a result, the first day of administration was set as the day 0. Figs. 21 and 22 show the state of tumors on the 5th and 10th days after administration of OX40L. Figs. 23 and 24 show the state of tumors on the 5th and 10th days after administration of 4-1BBL-PIV2-HN CT. Fig. 25 shows the average value of the tumor volumes at the administration site to which OX40L had been administered and the distant site. Fig. 26 shows the average values of the tumor volumes at the administration site to which 4-1BBL-PIV2-HN CT had been administered and the distant site with time.

The tumor volume was acquired by calculating "minor axis x minor axis x major axis x 0.5", and the average of three animals was used for the tumor volume. An animal with a maximum tumor diameter of 15 mm was considered dead. In a case where the number of measurable animals reached one, the numerical value was excluded.

In the present test, in regard to the antitumor effects of BC-PIV/OX40L + LPS or BC-PIV/4-1BBL-PIV2-HN CT + LPS, it was confirmed that suppression of tumor proliferation was strongly inhibited at both sites of the administration site and the distant site due to the administration of the anti-asialo GM-1 antibody or anti-CD4/8 antibody (see group 9 of Figs. 21 and 22 (administration of anti-asialo GM1 antibody, 4 days or 10 days later), see group 10 of Figs. 23 and 24 (administration of anti-asialo GM1 antibody, 4 days or 10 days later), see group 11 of Figs. 21 and 22 (administration of anti-CD4/8 antibody, 4 days or 10 days later), see group 12 of Figs. 23 and 24 (administration of anti-CD4/8 antibody, 4 days or 10 days later).

As can be seen from the state of the tumors in the group 7, group 9, and group 11 on the 10th day from the administration in Fig. 22, the tumors were clearly increased in the group 9 (NK cell inhibitory antibody was added to administration group 7) and the group 11 while the tumors were likely to shrink at the administration site and the distant site of the group 7 to which BC-PIV/OX40L + LPS had been administered. In the group 11 (CD4/8-positive T cell inhibitory antibody was added to administration group 7), the tumor cells metastasized into the abdominal cavity in two out of three mice, and the entire abdomen was rapidly enlarged. In the group 9, tumor growth at the administration site was significant. Regression or suppression of tumor growth only at the two tumor administration sites (administered to the right, left, and left tumors from the left animal) but also at the distant tumors of two tumors of three mice in the group 7 was significant as compared to the groups 9 and 11.

The same results were shown in the group 8, the group 10 (NK cell inhibitory antibody was added to administration group 8), and the group 12 (CD4/8-positive T cell inhibitory antibody was added to administration group 8) to which BC-PIV/4-1BBL-PIV2-HN CT + LPS had been administered, as shown in Fig. 24.

Further, even in the results of the tumor volumes in Figs. 25 and 26, in regard to the antitumor effects of BC-PIV/OX40L + LPS or BC-PIV/4-1BBL-PIV2-HN CT + LPS, it was found that suppression of tumor proliferation was strongly inhibited at both sites of the administration site and the distant site due to the administration of the anti-asialo GM1 antibody or anti-CD4/8 antibody.

Based on the results, it was suggested that the high antitumor effect of BC-PIV/OX40L + LPS or BC-PIV/4-1BBL-PIV2-HN CT + LPS was strongly related to NK cells and CD4-positive T cells/CD8-positive T cells. Previously, it was reported that the LPS strongly induces the maturation of dendritic cells (see Patent Document 3), and the activation of dendritic cells is also considered to be involved in the suppression of tumor proliferation.

Next, in regard to BC-PIV/OX40L + LPS (group 7) and BC-PIV/OX40L (group 5) in Figs. 21 and 22, tumors including distant tumors finally disappeared from one of three mice in both groups. At the administration site of the BC-PIV/OX40L administration group, the tumor grew once after three times of administrations, but then the tumor turned to shrinkage. That is, the antitumor effect was exhibited some time after administration. Since CTL occurred approximately one week after the immunity-eliciting operation and the effect was not recognized due to the inhibition of CD4/8-positive T cells, CTL was considered as the main factor of the shrinkage of the tumor. Further, as can be seen from the photograph of the group 7 in Fig. 21, multiple tumors in the administration site of the BC-PIV/OX40L + LPS administration group were turned black and necrotic and most of the tumors shrank immediately after the administration. Therefore, it was considered that death of tumor cells due to TNFα and NK cells induced by LPS occurred at the initial stage of administration. The tumors in the photograph after 10 days from administration appeared large at the distant sites in the BC-PIV/OX40L administration group, but the tumors did not grow and tended to regress with time. It was considered that systemic antitumor immunity, mainly CTL, acted on tumor cells at distant sites. On the contrary, the tumors at the distant sites in the BC-PIV/OX40L + LPS group tended to increase more than the case of BC-PIV/OX40L with time.

Here, a combination of the OX40 agonist antibody and LPS was set in the group 4. Based on the comparison between the group 4 and the group 7, the effects of the anti-OX40 agonist antibody or BC-PIV/OX40L combined with LPS were almost the same as each other, or BC-PIV/OX40L exhibited a more excellent effect. Here, it was suggested that LPS also has the ability to induce OX40 receptors on T cells. Based on the results of Fig. 17, it was reconfirmed that the OX40 receptor was induced by incorporating OX40L onto the envelope of BC-PIV, and the TNFRSF signal was efficiently activated. Similar effects were recognized even in BC-PIV/4-1BBL-PIV2-HN CT, into which 4-1BBL-PIV2-HN CT had been introduced, performed in parallel.

As a result, it was confirmed that a high antitumor effect, which was considered to be difficult to exhibit by administration of OX40L and 4-1BBL protein alone, was able to be exhibited to the same extent as that of the anti-OX40 agonist antibody by incorporating OX40L and/or 4-1BBL onto the envelope of BC-PIV, and a high antitumor effect was exhibited in forms other than the OX40 agonist antibody and the 4-1BB agonist antibody.

### [Experimental Example 10]

### (Antitumor effect of BC-PIV prepared by introducing 4-1BBL-PIV2-HN CT gene into Mlul site of plasmid for virus preparation on melanoma (B16 mouse melanoma cells))

BC-PIV/OX40L or BC-PIV/4-1BBL-PIV2-HN CT and/or LPS were found to have antitumor effects on mouse colorectal cancer cells (CT26). Therefore, the presence or absence of antitumor effects on other tumors (melanoma) was also examined.

The back of the B6 mouse was depilated, and 5×10⁵ B16 mouse melanoma cells were transplanted into two sites in the back SC region under anesthesia. Here, many mice had one site with tumor growth (there were multiple mice in which tumors appeared at different sites during the second administration), and a combination of BC- PIV/4-1BBL-PIV2-HN CT and LPS was administered to the tumors of mice with a tumor diameter of 4 to 7 mm.

The administered viral load was set to 5.9×10⁷ particles per mouse, the amount of LPS administered was set to 7.5 µg, and the administration was performed three times. The number of mice in one group was set to three. The dose was set as 60 µL.

Group 1: BC-PIV administration group
Group 2: BC-PIV/4-1BBL-PIV2-HN CT administration group
Group 3: BC-PIV/4-1BBL-PIV2-HN CT + LPS (7.5 µg) administration group

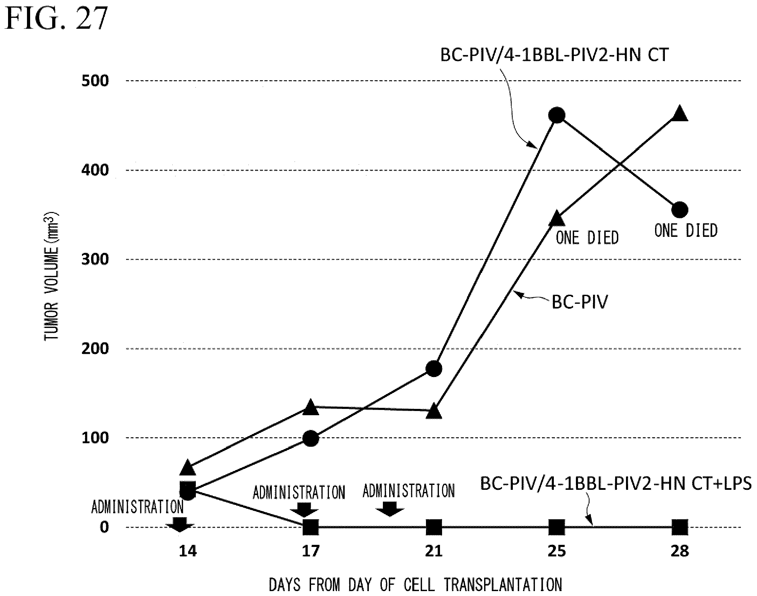

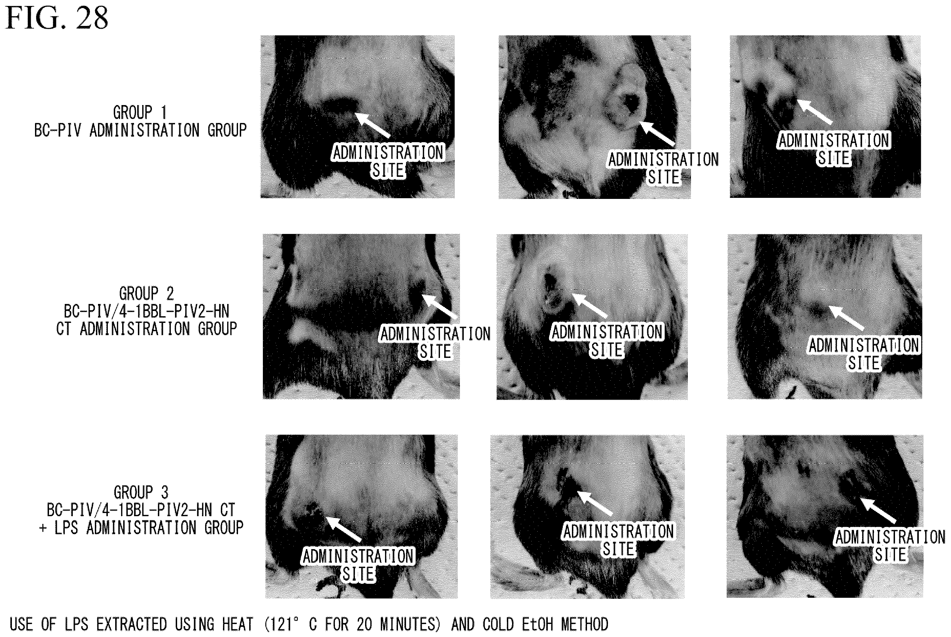

The average tumor volume of three mice (cases of dead mice were excluded) is shown in Fig. 27. Unlike CT26 colorectal cancer tumors, B16 melanoma was in a liquid state in the tumor, and the contents were often found to flow out in a case of the sample administration. In a case of the B16 melanoma, in the group 3 which was the BC-PIV/4-1BBL-PIV2-HN CT + LPS (7.5 µg) group, the tumor regression time was earlier than that of CT26 colorectal cancer, and the cancer was discolored and regressed (the rate of regression was faster than the rate in the case of CT26 colorectal cancer) 2 days after administration. Fig. 28 shows the state 3 days after the administration. In the group 3, the cancer at the administration site completely regressed. The proliferation of tumors on a side opposite to the administration side of the mice in this group was recognized after administration, but tumors of two mice proliferated a diameter of approximately 3 mm and regressed, and the tumors of both mice disappeared completely.

Since it has been pointed out that innate immunity by NK cells is also important for the antitumor effect of melanoma cells in addition to CTL, activation of NK cells due to LPS in addition to CTL induction of BC-PIV/4-1BBL-PIV2-HN CT was also considered to be important.

### [Experimental Example 11]

### (Antitumor effect of BC-PIV prepared by introducing OX40L or 4-1BBL-PIV2-HN CT gene into NotI site of plasmid for virus preparation)

As shown in Figs. 21, 22, and 25, the tumor volume at the administration site was increased once after the final administration and then turned to shrinkage by administration of only BC-PIV/OX40L in which the OX40L gene was introduced into the Mlul site of the plasmid for virus preparation. In a case of tumors at the distant site, the proliferation of tumors was suppressed after administration.

The gene expression of BC-PIV shows a polar effect, and the amount of the NP gene product upstream of the plasmid for BC-PIV virus preparation is the maximum, and the amount of the gene product decreases toward the L gene downstream. Therefore, the expression level of the transgene is higher in BC-PIV in which the foreign gene is introduced into the NotI site on the 5'upstream side of the plus chain than the Mlul site of the plasmid for virus preparation. Therefore, the antitumor effect was investigated using BC-PIV in which the OX40L or 4-1BBL-PIV2-HN CT gene was introduced into the NotI site.

The animal model and the number of times and intervals of administration were the same as in the previous tests. PBS, BC-PIV (2.1×10⁷ particles), BC-PIV/OX40L (NotI) (2.1×10⁷ particles), and BC-PIV/4-1BBL-PIV2-HN CT (NotI) (2.1×10⁷ particles) were administered 3 times.

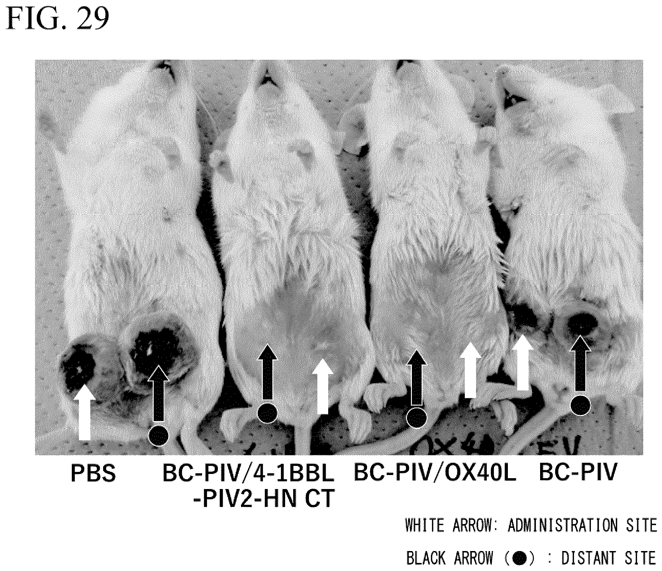

The results of proliferation of tumors 23 days after the first administration are shown in Fig. 29. In the administration of PBS and BC-PIV, the tumors at the administration site and the distant site proliferated, and the antitumor effect was not recognized. On the contrary, in the mice to which BC-PIV/OX40L and BC-PIV/4-1BBL-PIV2-HN CT had been administered, the tumors were completely regressed at both the administration site and the distant site 23 days after the start of administration, as shown in the figure. It was found that the antitumor effect increases as the amount of the TNFSF ligand protein mounted on BC-PIV increases. Further, the regression effect was exhibited on the tumors at the administration site and distant site without using LPS of Pantoea agglomerans.

### (Examination of antitumor effect of BC-PIV carrying two TNFSF ligand genes (antitumor effect of BC-PIV carrying two genes of OX40L and 4-1BBL))

Here, each of five kinds of TNFSF ligand genes was introduced into BC-PIV. Not all the five kinds of TNFRSF signals have the same characteristics as each other, and it is considered that the antitumor effects thereof are also different from each other. Therefore, two kinds of TNFSF ligands were introduced into one BC-PIV and the effects thereof were examined.

The amount of each ligand protein to be mounted on BC-PIV into which two genes were introduced is shown in the Western blotting of Fig. 9.

The preparation of the cancer-bearing mice, the dosage volume, the number of times of administration, and the like were set according to the methods as described above. The administration groups are described below.
Group 1: BC-PIV administration group
Group 2: BC-PIV/OX40L (NotI)·4-1BBL (Mlul) administration group
Group 3: BC-PIV/4-1BBL (NotI)·OX40L (Mlul) administration group
Group 4: BC-PIV/4-1BBL-PIV2-HN CT (NotI)·OX40L (Mlul) administration group

Each sample was prepared in an amount of 50 µL and administered. Here, the administered viral load was set to 1.6×10⁶ particles, which was smaller than usual, and the amount of virus was set such that the antitumor effect was not able to be obtained at a high level by a single dose of administration. The administration method was the same as described above.

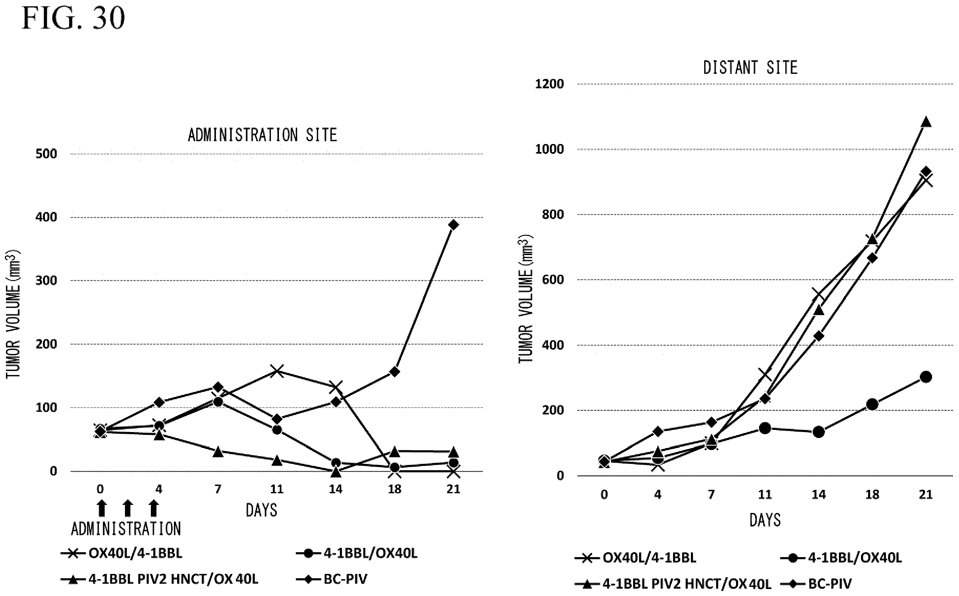

The results are shown in Fig. 30. The amounts of ligand proteins incorporated into BC-PIV into which two ligands had been introduced were different from each other as shown in Fig. 9. Based on the results of the present test, the combination with a high expression level of 4-BBL had the tumor inhibitory effect, and the highest effect was recognized in the BC-PIV/4-1BBL (NotI)·OX40L (Mlul) administration group of the group 3.

However, since the synergistic effect was not high except for the combination of OX40L and 4-1BBL, the expression of GITRL, CD27L, and CD30L was confirmed, and the antitumor effect of the combination of GITRL and OX40L or 4-1BBL was examined.

### (Examination of antitumor effect of GITRL, CD27L, and CD30L and antitumor effect by two ligand proteins (GITRL/OX40L and GITRL/4-BBL))

The antitumor effect of BC-PIV/GITRL, BC-PIV/CD27L, or BC-PIV/CD30L carrying GITRL, CD27L, and CD30L and the antitumor effect of BC-PIV carrying two proteins, GITRL and OX40L or GITRL and 4-1BBL, were examined.

In the experiment, the transplant of CT26 colorectal cancer cells into the mice and the administration to the tumors were performed in the same manner as described above. After depilation of the ventral side of the mouse, 5×10⁵ mouse CT26 colorectal cancer cells were inoculated into two ventral subcutaneous (SC) sites under anesthesia. Six days later, animals whose major axis of tumor reached 5 to 7 mm were divided into the following groups (3 animals for each group), and the sample was administered to one of the two cancer sites. The administration was performed 3 times every other day.

The administered viral load was set to BC-PIV: 9.0×10⁶ particles, BC-PIV/GITRL: 9.0×10⁶ particles, BC-PIV/CD27L: 9.0×10⁶ particles or BC-PIV/CD30L: 9.0×10⁶ particles, BC-PIV/GITRL (NotI)·OX40L (Mlul): 2.4×10⁶ particles, BC-PIV/GITRL (NotI)·4-1BBL (Mlul): 2.4×10⁶ particles per mouse. The number of administered particles of BC-PIV into which two proteins were introduced was approximately 1/4 of the number of viruses compared to other cases.

The preparation of the cancer-bearing mice, the dosage volume, and the administration interval were set according to the methods as described above, and the administration group was as follows.
Group 1: PBS administration group
Group 2: BC-PIV administration group
Group 3: BC-PIV/GITRL administration group
Group 4: BC-PIV/CD30L administration group
Group 5: BC-PIV/CD27L administration group
Group 6: BC-PIV/GITRL (NotI) ·OX40L (Mlul)
Group 7: BC-PIV/GITRL (NotI)·4-1BBL (Mlul)

Samples were prepared and administered in an amount of 60 µL for the groups 1 to 5 and an amount of 50 µL for the groups 6 and 7.

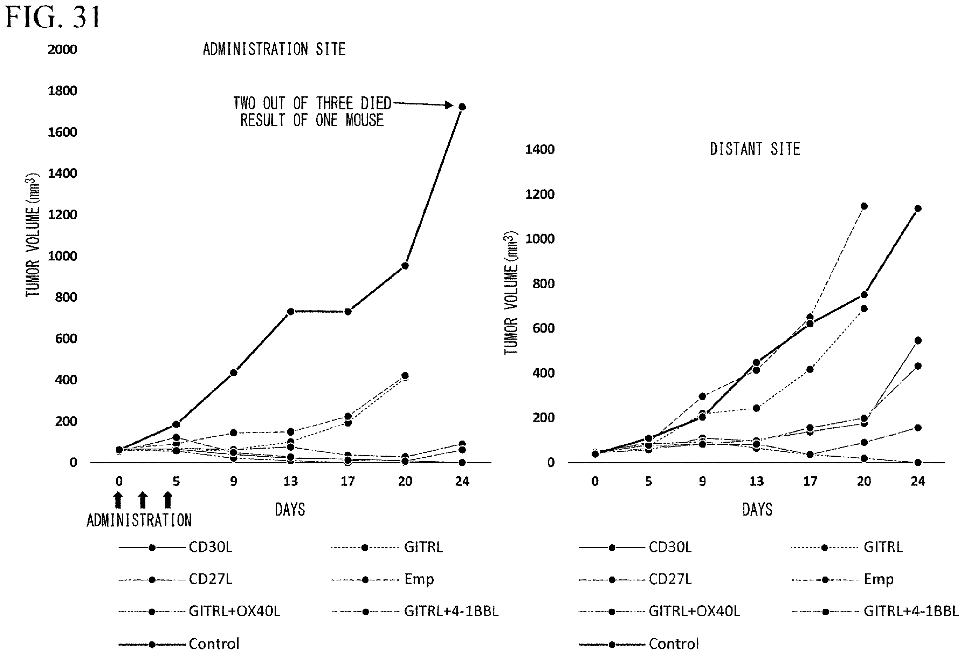

The results of changes in tumor volume with time are shown in Fig. 31. The average value of two or three mice was used as the tumor volume. The tumor diameter was measured on the day shown in the figure from the day of the first administration. From the third administration, the tumor diameter was measured every 3 days or 4 days, and the tumor volume was determined by calculating "minor axis × minor axis × major axis × 0.5". Animals whose major axis of tumor reached approximately 15 mm were treated as ethically dead.

As can be seen in the figure, in each administration group of BC-PIV carrying GITRL, CD27L, and CD30L, the GITRL was slightly inferior, but the antitumor effect was recognized at the administration site and the distant site as compared with the untreated group and the BC-PIV administration group.

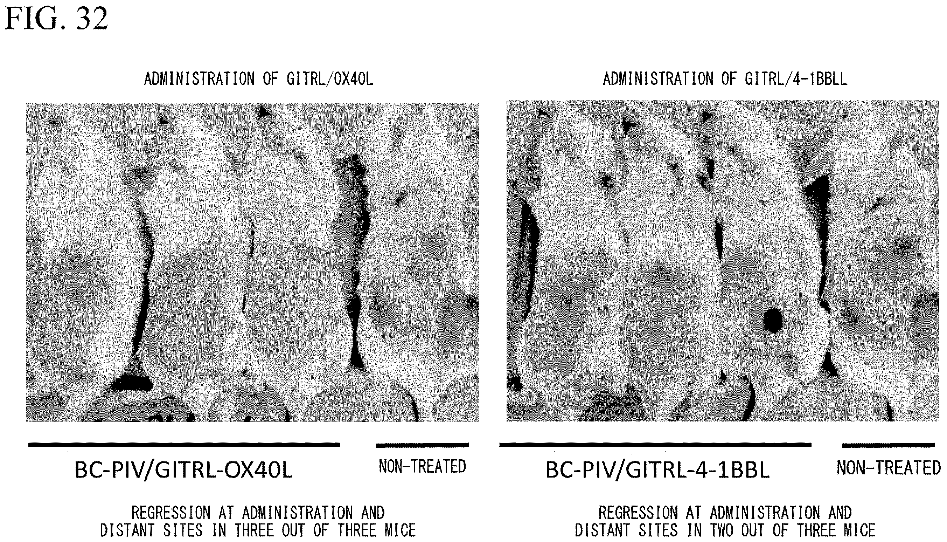

In addition, in the combination administration group of GITRL and OX40L or GITRL and 4-1BBL, the administered viral load is as small as approximately 1/4 of the viral load in the case of single introduction, and a high antitumor effect was recognized at the administration site and the distant site (24 days after the first administration: see Fig. 32) even though the effect with OX40L or 4-1BBL alone was not expected to be high based on the viral load. After 24 days, two out of three mice died in the untreated control, but surprisingly, the tumors at the administration site and the distant site were regressed in three out of three mice of the GITRL/OX40L administration group. In the mice of the GITRL/4-1BBL administration group, the tumors at the administration site and the distant site were regressed in two out of three mice.

As a result, it was shown that BC-PIV carrying two ligands may exhibit a higher antitumor effect than in a case where BC-PIV carries a TNFSF ligand alone.

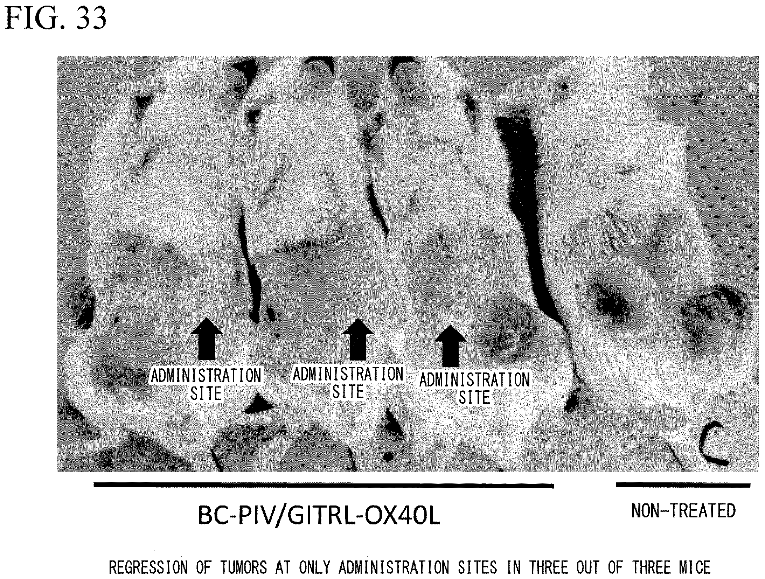

In the CD30L administered mice of the present test, tumors at the administration site were regressed in three out of three mice, and only the tumors at the distant site were increased as shown in Fig. 33.

### (Examination of antitumor immune effect using inactivated BC-PIV)

Adverse events of hepatotoxicity have been reported in clinical tests using agonist antibodies (anti-human 4-BBL) that activate TNFRSF. The use of BC-PIV into which TNFSF ligand has been introduced is devised to reduce such risk. Furthermore, from the viewpoint of enhancing the safety, the antitumor effect was examined using inactivated BC-PIV. Inactivation of BC-PIV was carried out in accordance with Japanese Patent No. 6358706.

Efficacy evaluation of inactivated BC-PIV was performed using BC-PIV into which OX40L or 4-1BBL had been introduced.
The combinations are described below.
Group 1: PBS administration group
Group 2: BC-PIV administration group
Group 3: Inactivated BC-PIV administration group
Group 4: BC-PIV/OX40L administration group
Group 5: Inactivated BC-PIV/OX40L administration group
Group 6: BC-PIV/4-1BBL administration group
Group 7: Inactivated BC-PIV/4-1BBL administration group

The test was performed in the same manner as described above, and the number of transplanted cells was set to 5×10⁵.

The administered viral load was set to (inactivated) BC-PIV: 3.0×10⁷ particles, (inactivated) BC-PIV/OX40L: 3.0×10⁷ particles, (inactivated) BC-PIV/4-1BBL: 3.0×10⁶ particles. The number of particles of (inactivated) BC-PIV/4-1BBL was set to 1/10 of the amounts of other cases. The administration method was the same as a method of the related art. As the CT26 cells used here, CT26 cells different from those of the related art, and the sensitivity to the antitumor immune agent was higher than that of CT26 cells of the related art.

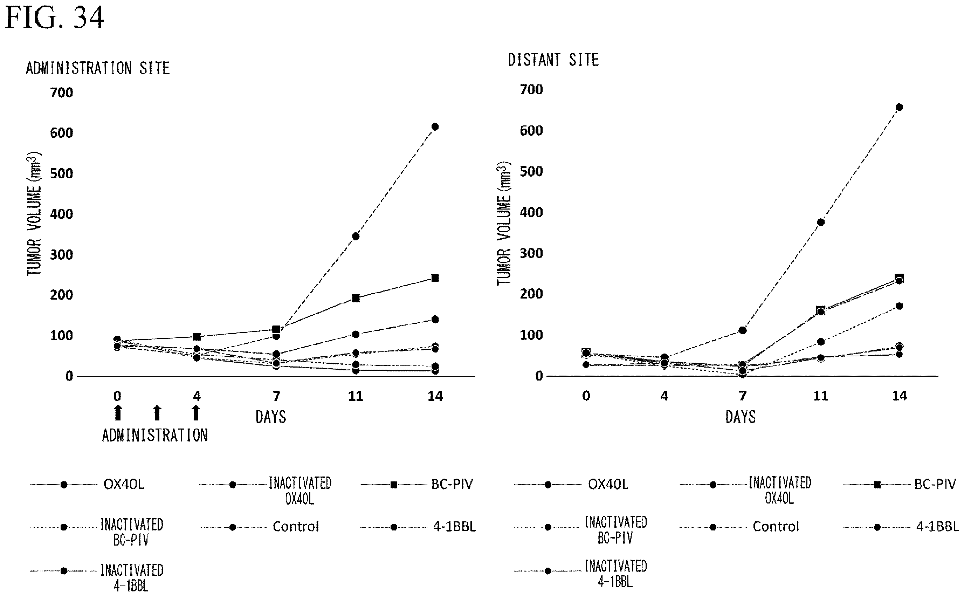

The results are shown in Fig. 34. In regard to OX40L, the antitumor effect was recognized in the untreated group regardless of the inactivation as shown in the figure. In regard to 4-1BBL, the dose was 1/10 of that of OX40L, but the effect on the CT26 tumor was recognized, and a higher antitumor effect was exhibited in the inactivated BC-PIV/4-1BBL administration group compared to the BC-PIV/4-1BBL administration group.

As a result, in BC-PIV including TNFSF ligand, it was found that both non-proliferative and inactivated BC-PIV effectively bind the TNFSF ligand to the TNFSF receptor and efficiently transmit signals, and exhibit the antitumor effect.

### [Experimental Example 12]

### (Incorporation of human OX40L into enveloped virus other than hPIV2)

### (Incorporation of human OX40L into RSV virus)

BC-PIV has an enveloped structure derived from human parainfluenza virus type 2 (hPIV2). The incorporation of human OX40L in viruses with enveloped structures other than hPIV2 was investigated. Since it was confirmed in previous tests that all of mouse and human OX40L, 4-1BBL, GITRL, CD27L, and CD30L were incorporated into the envelope membrane of BC-PIV, it was considered that the incorporation of the TNFSF ligand protein into other envelope viruses was able to be made in a case where one TNFSF ligand protein was incorporated into the envelope. Therefore, the incorporation of human OX40L, as a representative example, into other virus envelopes was examined.

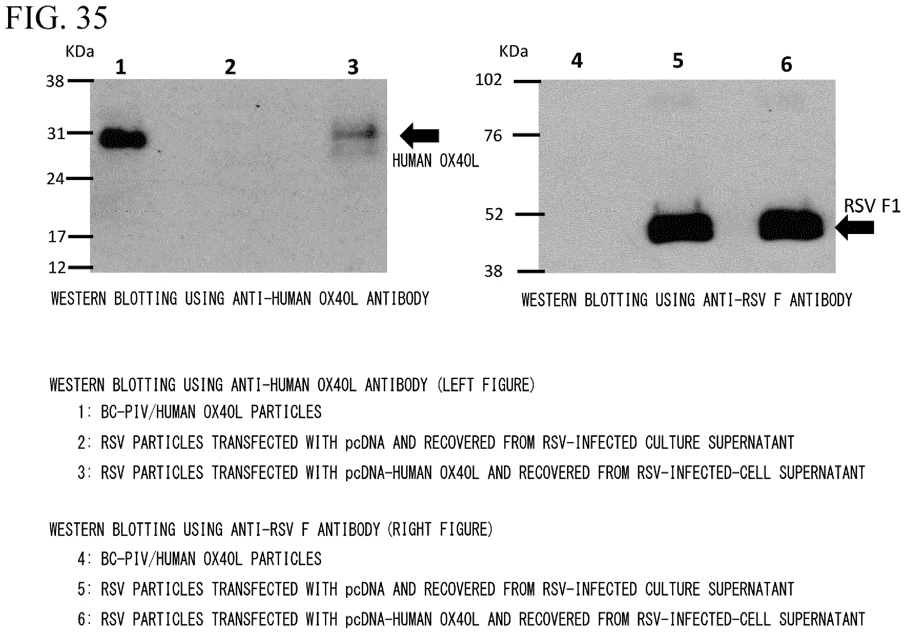

According to the method, cells were transfected with a plasmid carrying the human OX40L gene, cells expressing human OX40L were infected with a virus having the enveloped structure, the virus was recovered from the culture supernatant, and the incorporation of the human OX40L protein into the virus particles was evaluated by Western blotting (see Figure 35).

The Long strain of RS virus was used as the virus having an enveloped structure, and HEp2 cells were used as the infected cells. The human OX40L gene was introduced into a pcDNA (registered trademark) 3.1 plasmid (thermofisher). The plasmid (3 µg) was used to transfect into HEp2 cells (9×10⁵ cells). As a negative control, a pcDNA3.1 plasmid in which the human OX40L gene had not been inserted was transfected into HEp2 cells. Two days later, each cell was infected with the same amount of RS virus. Two days after RS virus infection, since HEp2 cells exhibited a cytopathic effect (CPE) due to the virus, the culture supernatant was recovered. The preparation of virus particles from the culture supernatant was carried out in conformity with Example 2. An anti-human OX40L antibody (ProSci, cat #: 7243) was used for Western blotting. BC-PIV including human OX40L was used as a positive control.

As shown in Fig. 35, lanes 1 and 4 are human OX40L-carrying BC-PIV particles, lanes 2 and 5 are RS virus particles transfected with a pcDNA3.1 plasmid and recovered from RS virus-infected cells, and lanes 3 and 6 are RS virus particles transfected with the human OX40L gene-carrying pcDNA3.1 plasmid and recovered from RS virus-infected cells. In the figure, lanes 1 to 3 show Western blotting using the anti-human OX40L antibody, and lanes 4 to 6 show Western blotting using the anti-RS virus F protein antibody. In lanes 5 and 6, a positive band was recognized in an anti-RS virus F antibody (Abcam cat #: ab43812), and RS virus was confirmed. Positive bands were confirmed in the anti-human OX40L antibody in the lane 1 containing positive control of human OX40L-carrying BC-PIV2 and the lane 3 containing RS virus recovered from RS virus-infected cells by transfection with human OX40L gene-carrying pcDNA3.1 plasmid.

These results indicate that the human OX40L protein is incorporated onto the envelope of RS virus.

### (Incorporation of human OX40L into PIV5)

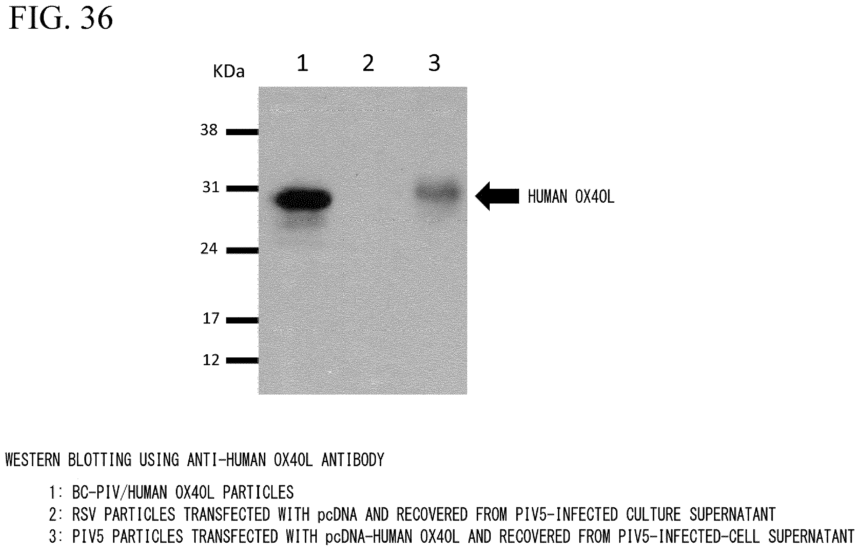

Further, a similar test was performed using PIV5 (former name SV5) virus as a virus having another enveloped structure and BHK cells as infected cells. The results are shown in Fig. 36. The lane 1 in the figure is human OX40L-carrying BC-PIV of a positive control, the lane 2 is PIV5 recovered from cells transfected with the pcDNA3.1 plasmid, and the lane 3 is PIV5 virus particles transfected with the human OX40L gene-carrying pcDNA3.1 plasmid, infected with PIV5 virus 24 hours after the transfection, and recovered from the culture supernatant 2 days after the infection. In the lane 1 containing a positive control for human OX40L-carrying BC-PIV2 and the lane 3 containing PIV5 transfected with the human OX40L gene-carrying pcDNA3.1 plasmid and recovered from PIV5 virus-infected cells, positive bands were confirmed in the anti-human OX40L antibody.

These results indicate that the human OX40L protein is incorporated onto the envelope of the PIV5 virus.

Based on the results described above, various kinds of enveloped viruses into which transiently expressed cells such as plasmids, constitutively expressed cells by recombination, and the TNF superfamily ligand proteins derived from recombinant viruses (the membrane anchor portion can be replaced with other proteins) were incorporated can be obtained. The possibility of antitumor immune agents using these viruses has been suggested.

### [Industrial Applicability]

According to the present invention, it is possible to provide an anticancer agent, a pharmaceutical composition for cancer treatment, and a kit having excellent antitumor effects.

## Claims

1. An anticancer agent comprising:
a virus presenting a desired protein or peptide on a virus particle envelope, as an active ingredient.

2. The anticancer agent according to Claim 1,
wherein the virus presents at least one TNF receptor superfamily ligand on the virus particle envelope.

3. The anticancer agent according to Claim 2,
wherein the at least one TNF receptor superfamily ligand is OX40L, 4-1BBL, GITRL, CD27L, CD30L, or a variant thereof having an identical function.

4. The anticancer agent according to Claim 3,
wherein a cytoplasmic tail (CT) sequence of the OX40L, 4-1BBL, GITRL, CD27L, CD30L, or variant thereof having an identical function is replaced with a CT sequence derived from HN of the virus.

5. The anticancer agent according to any one of Claims 1 to 4,
wherein the virus is human parainfluenza virus type 2.

6. The anticancer agent according to Claim 5,
wherein the human parainfluenza virus type 2 is a non-proliferative type virus in which an F gene is deleted from a genome.

7. The anticancer agent according to Claim 5 or 6,
wherein the human parainfluenza virus type 2 has an inactivated genome.

8. A pharmaceutical composition for cancer treatment, comprising:
the anticancer agent according to any one of Claims 1 to 7.

9. The pharmaceutical composition for cancer treatment according to Claim 8, further comprising:
a second anticancer agent.

10. The pharmaceutical composition for cancer treatment according to Claim 9, wherein the second anticancer agent contains an immune activator and/or an immune checkpoint inhibitor.

11. The pharmaceutical composition for cancer treatment according to Claim 9 or 10,
wherein the second anticancer agent contains an anti-OX40 agonist antibody, an anti-4-1BB agonist antibody, an anti-GITR agonist antibody, an anti-CD27 agonist antibody, or an anti-CD30 agonist antibody.

12. The pharmaceutical composition for cancer treatment according to any one of Claims 8 to 11, further comprising:
a lipopolysaccharide (LPS) derived from Pantoea agglomerans.

13. The pharmaceutical composition for cancer treatment according to any one of Claims 8 to 12,
wherein the pharmaceutical composition is used for intratumoral administration.

14. A kit for use in cancer treatment, which is used simultaneously or sequentially in the treatment, the kit comprising:
a virus presenting a desired protein or peptide on a virus particle envelope; and
a second anticancer agent.

15. The kit according to Claim 14,
wherein the virus presents at least one TNF receptor superfamily ligand on the virus particle envelope.

16. The kit according to Claim 15,
wherein the at least one TNF receptor superfamily ligand is OX40L, 4-1BBL, GITRL, CD27L, CD30L, or a variant thereof having an identical function.

17. The kit according to Claim 16,
wherein a cytoplasmic tail (CT) sequence of the OX40L, 4-1BBL, GITRL, CD27L, CD30L, or variant thereof having an identical function is replaced with a CT sequence derived from HN of the virus.

18. The kit according to any one of Claims 14 to 17,
wherein the virus is human parainfluenza virus type 2.

19. The kit according to Claim 18,
wherein the human parainfluenza virus type 2 is a non-proliferative type virus in which an F gene is deleted from a genome.

20. The kit according to Claim 18 or 19,
wherein the human parainfluenza virus type 2 has an inactivated genome.

21. The kit according to any one of Claims 14 to 20,
wherein the second anticancer agent contains an immune activator and/or an immune checkpoint inhibitor.

22. The kit according to any one of Claims 14 to 21,
wherein the second anticancer agent contains an anti-OX40 agonist antibody, an anti-4-1BB agonist antibody, an anti-GITR agonist antibody, an anti-CD27 agonist antibody, or an anti-CD30 agonist antibody.

23. The kit according to any one of Claims 14 to 22, further comprising:
a lipopolysaccharide (LPS) derived from Pantoea agglomerans.

24. The kit according to any one of Claims 14 to 23,
wherein the kit is used for intratumoral administration.


SEQUENCE LISTING

<110>  BioComo Incorporation
       Mie University

<120>  Anticancer agent, pharmaceutical composition for cancer
       treatment, and kit

<130>  PC28660

<150>  JP2018-191262
<151>  2018-09-14

<150>  JP2019-030897
<151>  2019-02-22

<160>  10

<170>  PatentIn version 3.5

<210>  1
<211>  597
<212>  DNA
<213>  Mus musculus

<400>  1
atggaagggg aaggggttca acccctggat gagaatctgg aaaacggatc aaggccaaga      60

ttcaagtgga agaagacgct aaggctggtg gtctctggga tcaagggagc agggatgctt     120

ctgtgcttca tctatgtctg cctgcaactc tcttcctctc cggcaaagga ccctccaatc     180

caaagactca gaggagcagt taccagatgt gaggatgggc aactattcat cagctcatac     240

aagaatgagt atcaaactat ggaggtgcag aacaattcgg ttgtcatcaa gtgtgatggg     300

ctttatatca tctacctgaa gggctccttt ttccaggagg tcaagattga ccttcatttc     360

cgggaggatc ataatcccat ctctattcca atgctgaacg atggtcgaag gattgtcttc     420

actgtggtgg cctctttggc tttcaaagat aaagtttacc tgactgtaaa tgctcctgat     480

actctctgcg aacacctcca gataaatgat ggggagctga ttgttgtcca gctaacgcct     540

ggatactgtg ctcctgaagg atcttaccac agcactgtga accaagtacc actgtga        597


<210>  2
<211>  558
<212>  DNA
<213>  Homo sapiens

<400>  2
gtgaagatgg aaagggtcca acccctggaa gagaatgtgg gaaatgcagc caggccaaga      60

ttcgagagga acaagctatt gctggtggcc tctgtaattc agggactggg gctgctcctg     120

tgcttcacct acatctgcct gcacttctct gctcttcagg tatcacatcg gtatcctcga     180

attcaaagta tcaaagtaca atttaccgaa tataagaagg agaaaggttt catcctcact     240

tcccaaaagg aggatgaaat catgaaggtg cagaacaact cagtcatcat caactgtgat     300

gggttttatc tcatctccct gaagggctac ttctcccagg aagtcaacat tagccttcat 360

taccagaagg atgaggagcc cctcttccaa ctgaagaagg tcaggtctgt caactccttg 420

atggtggcct ctctgactta caaagacaaa gtctacttga atgtgaccac tgacaatacc 480

tccctggatg acttccatgt gaatggcgga gaactgattc ttatccatca aaatcctggt 540

gaattctgtg tcctttga 558

<210> 3
<211> 930
<212> DNA
<213> Mus musculus

<400> 3
atggaccagc acacacttga tgtggaggat accgcggatg ccagacatcc agcaggtact 60

tcgtgcccct cggatgcggc gctcctcaga gataccgggc tcctcgcgga cgctgcgctc 120

ctctcagata ctgtgcgccc cacaaatgcc gcgctcccca cggatgctgc ctaccctgcg 180

gttaatgttc gggatcgcga ggccgcgtgg ccgcctgcac tgaacttctg ttcccgccac 240

ccaaagctct atggcctagt cgctttggtt ttgctgcttc tgatcgccgc ctgtgttcct 300

atcttcaccc gcaccgagcc tcggccagcg ctcacaatca ccacctcgcc caacctgggt 360

acccgagaga ataatgcaga ccaggtcacc cctgtttccc acattggctg ccccaacact 420

acacaacagg gctctcctgt gttcgccaag ctactggcta aaaaccaagc atcgttgtgc 480

aatacaactc tgaactggca cagccaagat ggagctggga gctcatacct atctcaaggt 540

ctgaggtacg aagaagacaa aaaggagttg gtggtagaca gtcccgggct ctactacgta 600

tttttggaac tgaagctcag tccaacattc acaaacacag gccacaaggt gcagggctgg 660

gtctctcttg ttttgcaagc aaagcctcag gtagatgact ttgacaactt ggccctgaca 720

gtggaactgt tcccttgctc catggagaac aagttagtgg accgttcctg gagtcaactg 780

ttgctcctga aggctggcca ccgcctcagt gtgggtctga gggcttatct gcatggagcc 840

caggatgcat acagagactg ggagctgtct tatcccaaca ccaccagctt tggactcttt 900

cttgtgaaac ccgacaaccc atgggaatga 930

<210> 4
<211> 765
<212> DNA
<213> Homo sapiens

<400> 4
atggaatacg cctctgacgc ttcactggac cccgaagccc cgtggcctcc cgcgccccgc 60

gctcgcgcct gccgcgtact gccttgggcc ctggtcgcgg ggctgctgct gctgctgctg 120

ctcgctgccg cctgcgccgt cttcctcgcc tgcccctggg ccgtgtccgg ggctcgcgcc 180

tcgcccggct ccgcggccag cccgagactc cgcgagggtc ccgagctttc gcccgacgat 240

cccgccggcc tcttggacct gcggcagggc atgtttgcgc agctggtggc ccaaaatgtt 300

ctgctgatcg atgggcccct gagctggtac agtgacccag gcctggcagg cgtgtccctg 360

acggggggcc tgagctacaa agaggacacg aaggagctgg tggtggccaa ggctggagtc 420

tactatgtct tctttcaact agagctgcgg cgcgtggtgg ccggcgaggg ctcaggctcc 480

gtttcacttg cgctgcacct gcagccactg cgctctgctg ctggggccgc cgccctggct 540

ttgaccgtgg acctgccacc cgcctcctcc gaggctcgga actcggcctt cggtttccag 600

ggccgcttgc tgcacctgag tgccggccag cgcctgggcg tccatcttca cactgaggcc 660

agggcacgcc atgcctggca gcttacccag ggcgccacag tcttgggact cttccgggtg 720

acccccgaaa tcccagccgg actcccttca ccgaggtcgg aataa 765

<210> 5
<211> 522
<212> DNA
<213> Mus musculus

<400> 5
atggaggaaa tgcctttgag agaatcaagt cctcaaaggg cagagaggtg caagaagtca 60

tggctcttgt gcatagtggc tctgttactg atgttgctct gttctttggg tacactgatc 120

tatacttcac tcaagccaac tgccatcgag tcctgcatgg ttaagtttga actatcatcc 180

tcaaaatggc acatgacatc tcccaaacct cactgtgtga atacgacatc tgatgggaag 240

ctgaagatac tgcagagtgg cacatattta atctacggcc aagtgattcc tgtggataag 300

aaatacataa aagacaatgc ccccttcgta gtacagatat ataaaaagaa tgatgtccta 360

caaactctaa tgaatgattt tcaaatcttg cctataggag gggtttatga actgcatgct 420

ggagataaca tatatctgaa gttcaactct aaagaccata ttcagaaaaa taacacatac 480

tgggggatca tcttaatgcc tgatctacca ttcatctctt ag 522

<210> 6
<211> 600
<212> DNA
<213> Homo sapiens

<400> 6
atgacattgc atccttcacc catcacttgt gaatttttgt tttccacagc tctcatttct 60

ccaaaaatgt gtttgagcca cttggaaaat atgcctttaa gccattcaag aactcaagga 120

gctcagagat catcctggaa gctgtggctc ttttgctcaa tagttatgtt gctatttctt 180

tgctccttca gttggctaat ctttattttt ctccaattag agactgctaa ggagccctgt 240

atggctaagt ttggaccatt accctcaaaa tggcaaatgg catcttctga acctccttgc 300

gtgaataagg tgtctgactg gaagctggag atacttcaga atggcttata tttaatttat 360

ggccaagtgg ctcccaatgc aaactacaat gatgtagctc cttttgaggt gcggctgtat 420

aaaaacaaag acatgataca aactctaaca aacaaatcta aaatccaaaa tgtaggaggg 480

acttatgaat tgcatgttgg ggacaccata gacttgatat tcaactctga gcatcaggtt 540

ctaaaaaata atacatactg gggtatcatt ttactagcaa atccccaatt catctcctag 600

<210> 7
<211> 596
<212> DNA
<213> Mus musculus

<400> 7
gtgcagggat gccggaggaa ggtcgccctt gcccctgggt tcgctggagc gggaccgcgt 60

tccagcgcca atggccatgg ctgctgctgg tggtgtttat tactgtgttt tgctgttggt 120

ttcattgtag cggactactc agtaagcagc aacagaggct gctggagcac cctgagccgc 180

acacagctga gttacagctg aatctcacag ttcctcggaa ggaccccaca ctgcgctggg 240

gagcaggccc agccttggga aggtccttca cacacggacc agagctggag gagggccatc 300

tgcgtatcca tcaagatggc ctctacaggc tgcatatcca ggtgacactg gccaactgct 360

cttccccagg cagcaccctg cagcacaggg ccaccctggc tgtgggcatc tgctcccccg 420

ctgcgcacgg catcagcttg ctgcgtgggc gctttggaca ggactgtaca gtggcattac 480

agcgcctgac atacctggtc cacggagatg tcctctgtac caacctcacc ctgcctctgc 540

tgccgtcccg caacgctgat gagaccttct ttggagttca gtggatatgc ccttga 596

<210> 8
<211> 582
<212> DNA
<213> Homo sapiens

<400> 8
atgccggagg agggttcggg ctgctcggtg cggcgcaggc cctatgggtg cgtcctgcgg 60

gctgctttgg tcccattggt cgcgggcttg gtgatctgcc tcgtggtgtg catccagcgc 120

ttcgcacagg ctcagcagca gctgccgctc gagtcacttg ggtgggacgt agctgagctg 180

cagctgaatc acacaggacc tcagcaggac cccaggctat actggcaggg gggcccagca 240

ctgggccgct ccttcctgca tggaccagag ctggacaagg ggcagctacg tatccatcgt 300

gatggcatct acatggtaca catccaggtg acgctggcca tctgttcctc cacgacggcc 360

tccaggcacc accccaccac cctggccgtg ggaatctgct ctcccgcctc ccgtagcatc 420

agcctgctgc gtctcagctt ccaccaaggt tgtaccattg cctcccagcg cctgacgccc 480

ctggcccgag gggacacact ctgcaccaac ctcactggga cacttttgcc ttcccgaaac 540

actgatgaga ccttctttgg agtgcagtgg gtgcgcccct ga 582

<210> 9
<211> 724
<212> DNA
<213> Mus musculus

<400> 9
aagcatggag ccagggctgc aacaagcagg cagctgtggg gctccttccc ctgacccagc 60

catgcaggtg cagcccggct cggtagccag cccctggaga agcacgaggc cctggagaag 120

cacaagtcgc agctacttct acctcagcac caccgcactg gtgtgccttg ttgtggcagt 180

ggcgatcatt ctggtactgg tagtccagaa aaaggactcc actccaaata caactgagaa 240

ggcccccctt aaaggaggaa attgctcaga ggatctcttc tgtaccctga aaagtactcc 300

atccaagaag tcatgggcct acctccaagt gtcaaagcat ctcaacaata ccaaactgtc 360

atggaacgaa gatggcacca tccacggact catataccag gacgggaacc tgatagtcca 420

attccctggc ttgtacttca tcgtttgcca actgcagttc ctcgtgcagt gctcaaatca 480

ttctgtggac ctgacattgc agctcctcat caattccaag atcaaaaagc agacgttggt 540

aacagtgtgt gagtctggag ttcagagtaa gaacatctac cagaatctct ctcagttttt 600

gctgcattac ttacaggtca actctaccat atcagtcagg gtggataatt tccagtatgt 660

ggatacaaac actttccctc ttgataatgt gctatccgtc ttcttatata gtagctcaga 720

ctga 724

<210> 10
<211> 705
<212> DNA
<213> Homo sapiens

<400> 10
atggacccag ggctgcagca agcactcaac ggaatggccc ctcctggaga cacagccatg 60

catgtgccgg cgggctccgt ggccagccac ctggggacca cgagccgcag ctatttctat 120

ttgaccacag ccactctggc tctgtgcctt gtcttcacgg tggccactat tatggtgttg 180

gtcgttcaga ggacggactc cattcccaac tcacctgaca acgtccccct caaaggagga 240

aattgctcag aagacctctt atgtatcctg aaaagggctc cattcaagaa gtcatgggcc 300

tacctccaag tggcaaagca tctaaacaaa accaagttgt cttggaacaa agatggcatt 360

ctccatggag tcagatatca ggatgggaat ctggtgatcc aattccctgg tttgtacttc 420

atcatttgcc aactgcagtt tcttgtacaa tgcccaaata attctgtcga tctgaagttg 480

gagcttctca tcaacaagca tatcaaaaaa caggccctgg tgacagtgtg tgagtctgga 540

atgcaaacga aacacgtata ccagaatctc tctcaattct tgctggatta cctgcaggtc 600

aacaccacca tatcagtcaa tgtggataca ttccagtaca tagatacaag cacctttcct 660

cttgagaatg tgttgtccat cttcttatac agtaattcag actga 705